# EUROPÄISCHE PATENTSCHRIFT

(11) **EP 0 795 547 B1**
(45) Veröffentlichungstag und Bekanntmachung des Hinweises auf die Patenterteilung: **10.05.2000**
(21) Anmeldenummer: 97103163.8
(22) Anmeldetag: 27.02.1997
(51) Int. Cl.: C07D 215/26, A61K 31/47

(54) **(Aminoalkyl- und Acylaminoalkyl-oxy)benzyloxychinoline, Verfahren zu deren Herstellung und ihre Verwendung als Bradykinin-Rezeptorantagonisten**
(Aminoalkyl- and acylaminoalkyl-oxy)benzyloxyquinolines, process for their preparation and use as Bradykinin receptor antagonists
(Aminoalkyl- et acylaminoalkyl-oxy)benzyloxyquinolines, procédé de préparation et leur utilisation comme antagonistes des récepteurs de la bradykinine

(30) Priorität: 13.03.1996 DE 19609827
(43) Veröffentlichungstag der Anmeldung: 17.09.1997
(73) Patentinhaber: HOECHST AKTIENGESELLSCHAFT, 65929 Frankfurt am Main (DE)
(72) Erfinder: Heitsch, Holger Dr., 55252 Mainz-Kastel (DE); Wagner, Adalbert, Dr., 86368 Gersthofen (DE); Wirth, Klaus, Dr., 65830 Kriftel (DE); Schölkens, Bernward, Prof. Dr., 65779 Kelkheim (DE); Nölken, Gerhard, Dr., 65843 Salzbach (DE)

(56) Entgegenhaltungen:
- EP-A- 0 622 361
- WO-A-96/13485
- WO-A-96/40639
- WO-A-97/07115

## Beschreibung

Aus EP-A 622 361, US 5 212 182, US 5 216 165 und US 5 438 064 sind O- und N-substituierte Chinoline und ihre Verwendung als Bradykinin-Rezeptorantagonisten bekannt. Chinoline, die in Position 8 Substituenten mit einer Aminoalkylether- und eine Acylaminoalkylether-Funktion aufweisen, sind nicht beschrieben. In WO 96 13485, die eine ältere Anmeldung im Sinne von Art. 54(3) EPÜ ist, sind Pyridopyrimidone, Chinoline und kondensierte N-Heterozyklen als Bradykinin-Antagonisten beschrieben.

Die in der vorliegenden Anmeldung beschriebenen Aminoalkyl- und Acylaminoalkylether zeichnen sich durch eine hohe Affinität zum Bradykinin-B₂-Rezeptor und verbesserter Wasserlöslichkeit aus und werden durch die allgemeine Formel (I) dargestellt, 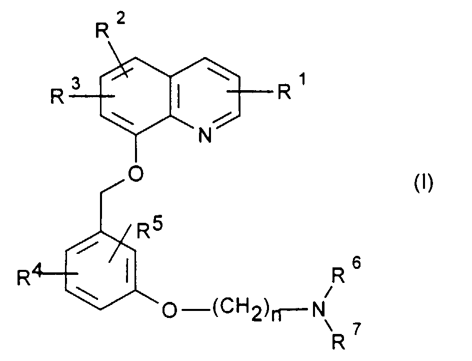 in welcher die Symbole die folgende Bedeutung haben:
- R¹,R²,R³: gleich oder verschieden
1. (C₁-C₅)-Alkyl,
2. (C₆-C₁₀)-Aryl,
3. (C₁-C₃)-Alkyl-(C₆-C₁₀)-Aryl,
4. Halogen,
5. Wasserstoff,
6. (C₃-C₈)-Cycloalkyl,
7. CHO,
8. CO-O-(C₁-C₃)-alkyl,
9. COOH;
- R⁴,R⁵: gleich oder verschieden
1. Wasserstoff,
2. Halogen,
3. (C₁-C₃)-Alkoxy,
4. Nitro,
5. Cyano,
6. S-(C₁-C₃)-Alkyl;
- n: eine Zahl von 1 bis 8;
- R⁶: 1. Wasserstoff,
2. (C₁-C₃)-Alkyl,
3. (C₃-C₅)-Alkylalkenyl,
4. (C₁-C₃)-Alkyl-(C₆-C₁₀)-Aryl;
- R⁷: Wasserstoff und die folgenden substituierten oder unsubstituierten Acylreste:
(C₁-C₆)-Alkanoyl (z.B. Formyl, Acetyl, Propionyl usw.), (C₁-C₃)-Alkoxy-(C₂-C₆)-alkanoyl (z.B. Methoxyacetyl, Ethoxyacetyl usw.), (C₁-C₆)-Alkylcarbamoyl-(C₂-C₆)-alkanoyl (z.B. Methylcarbamoylacetyl usw.), (C₆-C₁₂)-Aryl-(C₂-C₆)-alkanoyl (z.B. Phenylacetyl, Tolylacetyl usw.), (C₃-C₇)-Alkenoyl (z.B. Acryloyl, Crotonoyl usw.), (C₃-C₈)-Cycloalkylcarbonyl (z.B. Cyclopropylcarbonyl, Cyclohexylcarbonyl usw.), (C₅-C₇)-Cycloalkenylcarbonyl (z.B. Cyclohexenylcarbonyl usw.), (C₁-C₃)-Alkoxycarbonyl (z.B. Methoxycarbonyl, Ethoxycarbonyl usw.), (C₆-C₁₂)-Aryloxycarbonyl (z.B. Phenoxycarbonyl usw.), (C₆-C₁₂)-Aroyl (z.B. Benzoyl, Naphthoyl usw.), (C₁-C₃)-Alkoxy-(C₆-C₁₂)-aroyl (z.B. Methoxybenzoyl usw.), Halogen-(C₆-C₁₂)-aroyl (z.B. Chlorobenzoyl usw.), (C₆-C₁₂)-Aryl-(C₃-C₆)-alkenoyl (z.B. Cinnamoyl, Allocinnamoyl, α-Methylcinnamoyl, 4-Methylcinnamoyl usw.), (C₁-C₃)-Alkoxy-(C₆-C₁₂)-aryl-(C₃-C₆)-alkenoyl (z.B. Methoxycinnamoyl, Ethoxycinnamoyl, Dimethoxycinnamoyl usw.), (C₁-C₃)-Alkylendioxy-(C₆-C₁₂)-aryl-(C₃-C₆)-alkenoyl (z.B. Methylendioxycinnamoyl usw.), Nitro-(C₆-C₁₂)-aryl-(C₃-C₆)-alkenoyl (z.B. Nitrocinnamoyl usw.), Cyano-(C₆-C₁₂)-aryl-(C₃-C₆)-alkenoyl (z.B. Cyanocinnamoyl usw.), Halogen-(C₆-C₁₂)-aryl-(C₃-C₆)-alkenoyl (z.B. Chlorocinnamoyl, Dichlorocinnamoyl usw.), Halogen-(C₁-C₃)-alkyl-(C₆-C₁₂)-aryl-(C₃-C₆)-alkenoyl (z.B. Trifluoromethylcinnamoyl), Hetero-(C₃-C₈)-cycloalkyl-(C₆-C₁₂)-aryl-(C₃-C₆)-alkenoyl (z.B. Morpholinocinnamoyl usw.), Amino-(C₆-C₁₂)-aryl-(C₃-C₆)-alkenoyl (z.B. Aminocinnamoyl), (C₁-C₄)-Alkylamino-(C₆-C₁₂)-aryl-(C₃-C₆)-alkenoyl, (z.B. Methylaminocinnamoyl, Dimethylaminocinnamoyl usw.), (C₂-C₅)-Acylamino-(C₆-C₁₂)-arylcinnamoyl (z.B. Acetylaminocinnamoyl, Cyclopropylcarbonylaminocinnamoyl usw.), (C₁-C₃)-Alkoxycarbonylamino-(C₆-C₁₂)-arylcinnamoyl (z.B. Methoxycarbonylaminocinnamoyl usw.), (C₁-C₄)-Alkylaminocarbonylaminocinnamoyl (z.B. Ethylaminocarbonylaminocinnamoyl), Hetero-(C₆-C₁₂)-aryl-(C₂-C₆)-alkanoylamino-(C₆-C₁₂)-aryl-(C₃-C₆)-alkenoyl (z.B. Pyridylacetylaminocinnamoyl usw.), (C₆-C₁₂)-Aroylamino-(C₆-C₁₂)-aryl-(C₃-C₆)-alkenoyl (z.B. Benzoylaminocinnamoyl usw.), Hetero-(C₆-C₁₂)-arylcarbonylamino-(C₆-C₁₂)-aryl-(C₃-C₆)-alkenoyl (z.B. Pyridylcarbonylaminocinnamoyl usw.), (C₁-C₅)-Alkylsulfonylamino-(C₆-C₁₂)-aryl-(C₃-C₆)-alkenoyl (z.B. Ethylsulfonylaminocinnamoyl usw.), (C₁-C₅)-Alkylureido-(C₆-C₁₂)-aryl-(C₃-C₆)-alkenoyl (z.B. Ethylureidocinnamoyl usw.), (C₂-C₆)-Alkanoyl-(C₆-C₁₂)-aryl-(C₃-C₆)-alkenoyl (z.B. Acetylcinnamoyl), (C₁-C₅)-Alkoxycarbonyl-(C₆-C₁₂)-aryl-(C₃-C₆)-alkenoyl (z.B. Methoxycarbonylcinnamoyl usw.), (C₁-C₅)-Alkylcarbamoyl-(C₆-C₁₂)-aryl-(C₃-C₆)-alkenoyl (z.B. Ethylcarbamoylcinnamoyl usw.), (C₆-C₁₂)-Arylcarbamoyl-(C₆-C₁₂)-aryl-(C₃-C₆)-alkenoxyl (z.B. Phenylcarbamoylcinnamoyl usw.), (C₆-C₁₂)-Aryl-(C₁-C₅)-alkoxycarbonyl (z.B. Benzyloxycarbonyl usw.), (C₁-C₅)-Alkylcarbamoyl (z.B. Ethylcarbamoyl usw.), (C₆-C₁₂)-Arylcarbamoyl (z.B. Phenylcarbamoyl), (C₆-C₁₂)-Aroylcarbamoyl (z.B. Benzoylcarbamoyl usw.), (C₁-C₆)-Alkylsulfonyl (z.B. Mesyl, Ethylsulfonyl usw.), (C₆-C₁₂)-Arylsulfonyl (z.B. Phenylsulfonyl usw.), (C₆-C₁₂)-Aryl-(C₁-C₆)-alkylsulfonyl (z.B. Benzylsulfonyl usw.) und Phthaloyl (für R⁶ = R⁷),
sowie deren physiologisch verträgliche Salze.

Alkyl, Alkenyl und Alkinyl können geradkettig oder verzweigt sein. Entsprechendes gilt für davon abgeleitete Reste wie z.B. Alkoxy.

(C₆-C₁₂)-Aryl bedeutet vorzugsweise Phenyl, Naphthyl oder Biphenylyl. Entsprechend sind davon abgeleitete Reste, wie Aryloxy, Aralkyl oder Aroyl, zu formulieren.

Unter Heteroaryl sind Reste mit bis zu 9 C-Atomen zu verstehen, die einen monocyclischen oder bicyclischen aromatischen Ring bilden, in denen eine oder mehrere CH-Gruppen durch N, O und/oder S ersetzt sind. Dies sind z.B. Thienyl, Furanyl, Imidazolyl, Oxazolyl, Isoxazolyl, Pyridyl, Pyrimidyl, Indolyl, Chinolyl, Imidazopyridyl.

Halogen steht für Fluor, Chlor, Brom und Jod, vorzugsweise für Chlor.

Unter physiologisch verträglichen Salzen von Verbindungen der Formel (I) versteht man sowohl deren organische als auch anorganische Salze, wie sie in Remington's Pharmaceutical Sciences (A.R. Gennaro (Editor), Mack Publishing Co., Easton, PA, 17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind andere Salze mit Salzsäure, Schwefelsäure, Phosphorsäure, oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Bevorzugt sind Verbindungen der Formel (I), worin die Symbole folgende Bedeutung haben:
- R¹,R²,R³: gleich oder verschieden
1. Wasserstoff,
2. (C₁-C₃)-Alkyl;
- R⁴,R⁵: Halogen;
- R⁶: 1. Wasserstoff,
2. Methyl, Ethyl
3. Benzyl;
und die übrigen Reste und Variablen wie oben definiert sind.

Besonders bevorzugt sind Verbindungen der Formel (I), in der die Symbole folgende Bedeutung haben:
- R⁷: 1. Wasserstoff,
2. einen Acylrest, wie (C₂-C₆)-Alkanoyl, (C₆-C₁₂)-Aryl-(C₂-C₆)-alkanoyl, (C₆-C₁₂)-Aryl-(C₂-C₆)-cycloalkanoyl, (C₁-C₆)-Alkylaminocarbonyl, (C₃-C₇)-Alkenylaminocarbonyl, (C₁-C₃)-Alkoxycarbonyl, (C₆-C₁₂)-Aryl-(C₁-C₆)-alkylaminocarbonyl, (C₆-C₁₂)-Aryl-(C₃-C₆)-alkenoyl, (C₁-C₃)-Alkoxy-(C₆-C₁₂)-aryl-(C₃-C₆)-alkenoyl, Halogen-(C₆-C₁₂)-aryl-(C₃-C₆)-alkenoyl, Halogen-(C₁-C₃)-alkyl-(C₃-C₆)-alkenoyl, Amino-(C₆-C₁₂)-aryl-(C₃-C₆)-alkenoyl, (C₁-C₆)-Alkylamino-(C₃-C₆)-alkenoyl, und Hetero-(C₆-C₁₂)-aryl-(C₃-C₆)-alkenoyl und Phthaloyl (für R⁶ = R⁷);
und die übrigen Reste und Variablen wie oben definiert sind.

Ganz besonders bevorzugt sind Verbindungen der Formel (I), in der die Symbole folgende Bedeutung haben:
- R¹,R²,R³: gleich oder verschieden
1. Wasserstoff,
2. Methyl, Ethyl, Propyl;
- R⁴,R⁵: Chlor;
- n: 1 bis 4;
- R⁶: Wasserstoff;
- R⁷: 1. Wasserstoff,
2. (C₂-C₅)-Alkanoyl,
3. (C₃-C₅)-Alkenoyl,
4. (C₁-C₅)-Alkylaminocarbonyl,
5. (C₆-C₁₀)-Aryl-(C₁-C₃)-alkylaminocarbonyl,
6. (C₁-C₅)-Alkyloxycarbonyl,
7. (C₆-C₁₀)-Aryl-(C₁-C₃)-alkyloxycarbonyl,
8. (C₆-C₁₀)-Aryl-(C₃-C₇)-cycloalkylcarbonyl,
9. ein trans-Zimtsäurerest, dessen Phenylring substituiert ist mit bis zu 2 gleichen oder verschiedenen Resten aus der Reihe
   a) Wasserstoff,
   b) (C₁-C₃)-Alkyl,
   c) Amino,
   d) (C₁-C₃)-Mono- und -Dialkylamino,
   e) Halogen,
   f) (C₁-C₃)-Halogenalkyl,
   g) (C₂-C₅)-Acylamino und
   h) (C₁-C₃)-Alkoxy.

Die Erfindung betrifft weiterhin ein Verfahren zur Herstellung von Verbindungen der Formel (I), das dadurch gekennzeichnet ist, daß man
a) eine Verbindung der Formel (II) 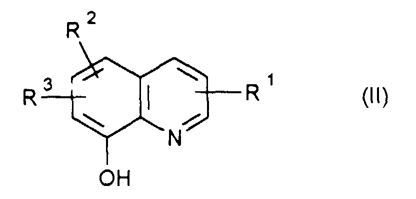 worin R¹, R² und R³ wie oben definiert sind, mit einer Verbindung der Formel (III) 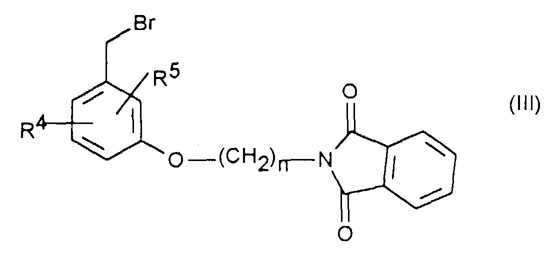 worin R⁴, R⁵ und n wie oben definiert sind, in Gegenwart von Metallhydriden, wie Lithium-, Kalium- oder Natriumhydrid, oder Alkalimetallcarbonaten, wie Natrium-, Kalium- oder Cäsiumcarbonat, in einem inerten Lösungsmittel, wie DMF oder DMSO, bei Temperaturen von 0°C bis 60 °C zu einer Verbindung der Formel (IV) 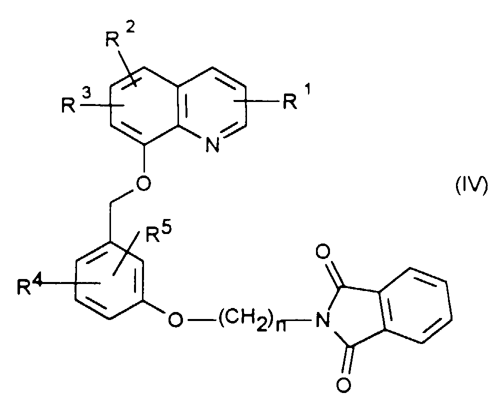 wobei die Symbole und Variablen wie oben definiert sind, umsetzt;
b) eine Verbindung der Formel (IV) durch Hydrazinolyse in Ethanol bei Rückfluß in eine Verbindung der Formel (Ia), 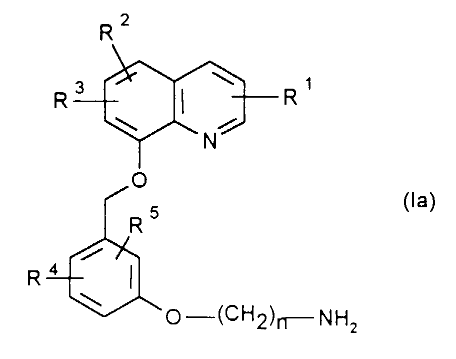 worin R¹, R², R³, R⁴, R⁵ und n wie oben definiert sind, überführt;
c) gegebenenfalls die Verbindungen der Formel (Ia) nach bekannten Methoden acyliert und/oder alkyliert, und
d) gegebenenfalls die erhaltenenen Verbindungen der Formel (I) nach bekannten Methoden in ihre physiologisch verträglichen Salze überführt.

Die Acylierung der Verbindungen der Formel (Ia) erfolgt durch Umsatz mit den entsprechend substituierten Carbonsäuren und Sulfonsäuren oder deren aktivierter Derivate und Isocyanaten.

Als aktivierte Säurederivate kommen dabei Säurechloride, Säureanhydride und Aktivester in Frage, z.B. Carbonsäurechloride und -bromide, gemischte Anhydride, symmetrische Anhydride, p-Nitrophenylester und Hydroxysuccinimidester. Die Wahl eines dieser aktivierten Derivate ist abhängig von der einzuführenden Acylgruppe.

Im Falle der freien Säuren erfolgt die Acylierung in Gegenwart der in der Peptidchemie angewandten Kondensationsreagenzien, siehe z.B. Houben-Weyl, Methoden der Organischen Chemie, Bd 15/2, Georg Thieme Verlag, Stuttgart 1974, insbesondere aber Carbodiimide wie z.B. N,N'-Dicyclohexylcarbodiimid, N,N'-Diisopropylcarbodiimid und N-Ethyl-N'-(3-dimethylaminopropyl)-carbodiimid oder Uroniumsalze wie O-[Cyan-(ethoxycarbonyl)methylenamino]-1,1,3,3-tetramethyluronium-tetrafluoroborat (TOTU) und O-Benzotriazol-1-yl-N,N,N',N'-tetramethyluronium-hexafluorophosphat (HBTU).

Die Acylierung bzw. die Aktivierung der Säurederivate erfolgt in konventionellen organischen Lösungsmitteln wie CH₂Cl₂, Dioxan, THF oder DMF. Die Acylierung wird in Gegenwart einer anorganischen oder organischen Base bei Temperaturen von 0°C bis Rückfluß durchgeführt.

Verfahren zur Herstellung der Verbindungen der Formel (II) sind u.a. bekannt aus H. Fiedler, J. Prakt. Chemie, Bd. 13, 1961, 86 ff.

Die Verbindungen der Formel (III) werden durch Halogenierung der entsprechenden Methylverbindungen der Formel (V) 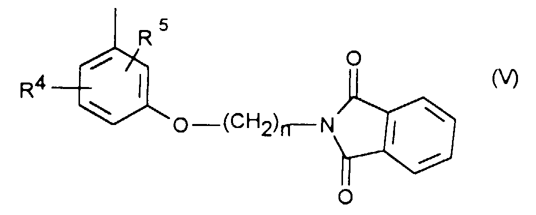 worin R⁴, R⁵ und n wie oben definiert sind, vorzugsweise mit N-Bromsuccinimid oder 1,3-Dibromo-5,5-dimethylhydantoin in Chlorbenzol unter Rückfluß dargestellt.

Die Herstellung der Verbindungen der Formel (V) erfolgt durch Alkylierung der Phenole der Formel (VI) 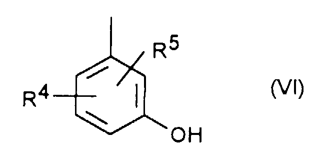 worin R⁴ und R⁵ wie oben definiert sind, mit kommerziell erhältlichen N-(Bromalkyl)-phthalimiden in Gegenwart von Alkalimetallcarbonaten wie Natrium-, Kalium- oder Cäsiumcarbonat in DMSO als Lösungsmittel bei Raumtemperatur und Reaktionszeiten von 10 min bis 1 h.

Die erfindungsgemäßen Verbindungen der Formel (I) haben einzeln oder in Kombination eine bradykinin-antagonistische Wirkung, die in verschiedenen Modellen getestet werden kann (siehe Handbook of Exp. Pharmacol. Vol. 25, Springer Verlag, 1970, S. 53-55), so z.B. am isolierten Rattenuterus, am Meerschweinchenileum, an der isolierten Pulmonalarterie des Meerschweinchens oder an der Jugularvene des Kaninchens.

Die Effekte der Verbindungen der Formel (I) auf die Bradykinin-induzierte Bronchokonstriktion und auf das Carregeenin-induzierte Pfotenödem können analog zu der in Br.J. Pharmacol., 102, 74-777 (1991) beschriebenen Vorgehensweise bestimmt werden.

Die Bestimmung der Affinität der Verbindungen der Formel (I) zum Bradykinin B₂ Rezeptor erfolgte an Membranpräparationen des Meerschweinchenileums (R.B. Innis et al., Proc. Natl. Acad. Sci. USA; 17 (1981) 2630) nach folgendem Verfahren:

### 1. Ligand: ³H-BRADYKININ (von NEN Du Pont)

### 2. Pufferansätze:

### a) TES-Puffer:

25 mM TES (SIGMA, Best.Nr.: T-4152)
1 mM 1,10-Phenanthrolin (SIGMA; Best.Nr.: P-9375)

### b) Inkubations-Puffer:

25 mM TES (SIGMA; Best.Nr.: T-4152)
1 mM1, 10-Phenanthrolin (SIGMA; Best.Nr.: P-9375)
0,1 % Albumin, Bovine (SIGMA; Best.Nr.: A-7906)
140 µg/ml Bacitracin (SIGMA; Best.Nr.: B-0125)
1 mM Dithiothreitol (SIGMA; Best.Nr.: D-0632)
1 µM Captopril - 1-[(2S)-3-Mercapto-2-methylpropionyl]-L-prolin

Beide Puffer werden mit 5 molarer NaOH auf pH 6,8 eingestellt.

### 3. Membranpräparation:

Meerschweinchen-Ilea werden durch vorsichtiges Ausstreichen grob vom Darm-Inhalt befreit und in 0,9%iger NaCI-Lösung gesäubert.

Die ca. 2 cm langen Ilea-Stücke werden in eiskalten TES-Puffer überführt (ca. 1 g /10 ml) und mit dem Ultraturrax ca. 30 sec. lang im Eisbad homogenisiert. Das Homogenat wird anschließend durch 3 Lagen Gaze gefiltert und das Filtrat bei 50.000 g 10 Minuten zentrifugiert.

Der Überstand wird verworfen, das Pellet in dem gleichem Volumen TES-Puffer rehomogenisiert und erneut bei 50.000 g 10 Minuten zentrifugiert. Das Pellet wird in Inkubations-Puffer rehomogenisiert (ca. 1 g/5 ml) und in Kryoröhrchen, 2 ml portioniert, bei -70°C eingefroren.

Die Proteinkonzentration der fertigen Membransuspension wird nach LO\NRY bestimmt und sollte ca. 15 µg /100 µl betragen.

### 4. Bindungstest:

Alle Inkubationen werden bei Raumtemperatur für 60 Minuten auf Mikrotiterplatten (96 x 300 µl) in 200 µl Volumen durchgeführt. Alle Ansätze in Inkubations-Puffer. Dazu werden 50 µl des Radioliganden, 50 µl des zu prüfenden Präparats und 100 µl der Membransuspension nacheinander in die Vertiefungen der Mikrotiterplatte pipettiert.

### a) Sättigungsexperimente (Heiße Sättigung):

Herstellung der ³H-Bradykinin-Lösung: Für die Sättigungsexperimente werden die Konzentrationen 0.05, 0.1, 0.2, 0.4, 0.6, 0.8, 1.0, 1.5, 2.0, 2.5 und 3.0 nMol/l, das entspricht 0.05 bis 3.0 pMol/ml, eingesetzt. Nach der Herstellung der entsprechenden Verdünnungen werden je 50 µl pro Probe vorgelegt.

Unspezifische Bindung: Für jede Konzentration des radioaktiven Liganden muß die unspezifische Bindung bestimmt werden. Dies kann durch Zugabe einer hohen Konzentration (1 -100 µMol) des nichtmarkierten Liganden, anderer Antagonisten oder Agonisten des Bradykinin-Rezeptors erreicht werden. In diesem Test wird HOE 140 (10 µMol/l) verwendet. Dazu werden 1,862 mg in 1 ml Dimethylsulfoxid (DMSO) gelöst, 1:25 mit Inkubationspuffer verdünnt und von dieser Lösung 50 µl zu den Proben in die Mikrotiterplatte gegeben. Die Reaktion wird durch die Zugabe von 100 µl der Membransuspension gestartet.

### b) Kompetitionsexperimente (IC₅₀):

Hier werden eine feste Größe des radioaktiven Liganden (0,25 bis 0,3 nMol/l ³H-Bradykinin) und verschiedene Konzentrationen der nichtmarkierten Agonisten oder Antagonisten eingesetzt.

Zu jeweils 50 µl der ³H-Bradykinin-Lösung werden 50 µl der zu prüfenden Präparate bzw. Standards in den Konzentrationen 10⁻⁵ bis 10⁻¹⁰ Mol/l zugegeben und die Reaktion durch Zugabe von 100 µl Membransuspension gestartet. Auch in diesem Test werden 3fach-Bestimmungen durchgeführt und drei Proben zur Bestimmung der unspezifischen Bindung mit 10 µMol/l HOE 140 inkubiert.

Die auf Kompetition zu prüfenden Präparate werden grundsätzlich in einer Konzentration von 1 mMol/l in Dimethylsulfoxid (DMSO) gelöst, und anschließend mit DMSO weiterverdünnt. Diese Lösung wird dann 1:25 mit Inkubationspuffer verdünnt.

Nach der Inkubation werden die Proben im Skatron Zellharvester über einen vorher mit 0, 1 % PEI (Polyethylenimin) angefeuchteten Whatmann GF/B Filterpapierstreifen abfiltriert und mit, je Probe, 10 ml eiskaltem TES-Puffer nachgewaschen. Die noch feuchten Filter werden in Mini-Scintillationsröhrchen ausgestanzt und mit 3 ml Scintillator aufgefüllt.

Nach ca. 12 Stunden Einweichzeit werden die Proben kurz aufgeschüttelt und im Beta-Counter gemessen.

### c) Screening:

Im Primär-Screening werden im allgemeinen nur 1-2 Konzentrationen des Prüfpräparats (10⁻⁵ und 10⁻⁶ Mol/l) eingesetzt. Ist bei der höchsten Konzentration eine Verdrängung des Radioliganden von 50% oder mehr nachweisbar, wird eine vollständige Analyse (Kompetitions-Experiment) mit mind. 8 Konzentrationen vorgenommen.

### 4. Auswertung:

Die Auswertung erfolgt mittels des LIGAND-Programmpakets (Mc Pherrson, Minson & Rodbard, Vertrieb: Elsevier-BIOSOFT), das die notwendigen Berechnungen zur Bestimmung von IC₅₀ und Kᵢ-Werten vornimmt. Dieses Programm führt außerdem grafische Darstellungen der Sättigungs- bzw. Verdrängungs-Kurven sowie den SCATCHARD-Plot, HILL-Plot oder HOFSTEE-Plot durch.

### 5. Testergebnisse

Nach dem oben angeführten Verfahren wurden für die Verbindungen der Beispiel 2, 5 und 6 als repräsentative Verbindungen der beschriebenen Aminoalkyl- und Acylaminoalkylether der Formel (I) folgende Kᵢ-Werte bestimmt:

| Beispiel | Kᵢ [nM] |
|---|---|
| 2 | 20 |
| 5 | 61 |
| 6 | 32 |

Desweiteren kann zur Bestimmung der bradykinin-antagonistischen Wirkung der Verbindungen der Formel (I) ihr Effekt auf die bradykinin-induzierte Kontraktion des Meerschweinchen-Ileums nach folgendem Protokoll gemessen werden:

Meerschweinchen im Gewicht von ca. 300 g (Morioth strain, ♂♀) werden durch Nackenschlag getötet und entblutet. Das Ileum wird in einer Länge von ca. 20 cm herauspräpariert, mit Tyrode-Lösung durchspült (Recordspritze) und so vom Darminhalt befreit. Nun wird es in 1,5 cm lange Abschnitte geteilt. Diese werden im 10 ml fassenden Organbädern, die mit Tyrode-Lösung gefüllt sind, fixiert und mit Dehnungsmeßstreifen (isometrische Kontraktionsmessung) verbunden. Die Vorlast beträgt 1 g. Die Tyrode-Lösung wird in einem Wasserbad auf 37°C erwärmt und mit Druckluft durchperlt.
Nach einem Intervall von 30 min. wird mit dem Versuch begonnen.
Nach Aufzeichnung der biologischen Null-Linie wird pro Organbad Bradykinin in einer Endkonzentration von 4 x 10⁻⁸ mol/l zugesetzt und die Konzentration aufgezeichnet. Danach wird 3 min. mit Tyrode-Lösung gespült und nach einer Ruhepause von 20 min. wieder Bradykinin hinzugefügt. Das Maximum der Kontraktion ist erreicht (Kontrolle). Wieder spülen, Ruhepause. Nun wird der Bradykinin-Antagonist zugefügt (Einwirkungszeit 10 min.). Danach wird wieder Bradykinin zugesetzt und die nun erfolgende Kontraktion mit der der Kontrolle verglichen. Die Aufzeichnung des Versuchs erfolgt auf einem Tintenschreiber.
Tyrode-Lösung (mM):
   - NaCl: 137
   - Glucose: 5,05
   - KCI: 2,68
   - NaHCO₃: 11,9
   - NaH₂PO₄: 0,47
   - MgCl₂ x 2H₂O: 0,49
   - CaCl₂ x 2H₂O: 0,68
Verstärker: TF6 V3 Fa. Fleck, Mainz
Tintenschreiber: Goerz Metrawatt SE 460, BBC
Bradykinin: Fa. Bachem

So besitzt z.B. die Verbindung gemäß Beispiel 1 den folgenden, nach obigem Verfahren ermittelten IC₅₀-Wert: IC₅₀ = 1.8 x 10⁻⁶ M.

Für die orale Anwendungsform oder zur Applikation auf die Schleimhäute werden die aktiven Verbindungen mit den dafür üblichen Zusatzstoffen wie Trägerstoften, Stabilisatoren oder inerten Verdünnungsmittein vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Die beschriebenen Verbindungen der Formel (I) und deren pharmakologisch geeigneten Salze sind potente Bradykinin-Antagonisten. Daher liegt ihr therapeutischer Nutzen in der Behandlung und/oder der Prävention aller pathologischer Zustände, die durch Bradykinin und Bradykinin-analoge Peptide vermittelt, ausgelöst oder unterstützt werden. Dies beinhaltet u.a. Allergien, Entzündungen, Autoimmunerkrankungen, Schock, Schmerz und, mehr speziell, Asthma, Husten, Bronchitis, Rhinitis, chronisch obstruktive Pulmonalerkrankungen, Pneumonitis, septischen Schock, endotoxischen Schock, anaphylaktischen Schock, disseminierenden intravaskulären Koagulopathie, Arthritis, Rheuma, Osteoarthritis, Lumbago, entzündungsinduzierte Knochenresorption, Konjunctivitis, Iritis, Kopfschmerz, Migräne, Zahnschmerz, Rückenschmerz, krebsbedingte Schmerzen, postoperativen Schmerz, Traumata (Wunden, Verbrennungen usw.), Ausschlag, Erytheme, Ödeme, Ekzeme, Dermatitis, Zoster, Herpes, Juckreiz, Psoriasis, Flechte, entzündliche Darmerkrankungen, Hepatitis, Pankreatitis, Gastritis, Ösophagitis, Nahrungsallergien, Geschwüre, Reizdarm, Angina, Hirnödem, niedriger Blutdruck, Thrombose, Schädel-Hirn- und Wirbelsäulen-Trauma, Frühgeburt, Atherosklerose, Aszites bei Malignom, Tumormetastasen, Hirnödem bei Tumoren Hitzeschädigung des Gehirns und Virus-Erkrankungen.

Da weiterhin bekannt ist, daß Bradykinin verknüpft ist mit der Freisetzung von Mediatoren, wie Prostaglandinen, Leukotrienen, Tachykininen, Histamin, Thromboxanen, besitzen die Verbindungen der Formel (I) somit auch das Potential zur Behandlung und/oder Prevention von den Krankheiten, die durch diese Mediatoren hervorgerufen werden.

Die Erfindung betrifft daher auch die Verwendung von Verbindungen der Formel (I) als Heilmittel und pharmazeutische Präparate, die diese Verbindungen enthalten.

Pharmazeutische Präparate und Heilmittel enthalten eine wirksame Menge des Wirkstoffs der Formel (I) - einzeln oder in Kombination - zusammen mit einem anorganischen oder organischen pharmazeutisch verwendbaren Trägerstoff.

Die Anwendung kann enteral, parenteral - wie z. B. subkutan, i.m. oder i.v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Sollten die Halbwertszeiten der beschriebenen Arzneistoffe in Körperflüssigkeiten unzureichend sein, ist der Einsatz von injizierbaren Retardzubereitungen sinnvoll.

Als Arzneiformen können z.B. ölige Kristallsuspensionen, Mikrokapseln, Rods oder Implantate verwendet werden, wobei die letzteren aus gewebsverträglichen Polymeren, insbesondere bioabbaubaren Polymeren, wie z.B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01-5 mg/l, bei systemischen Applikationsformen sind 0,01-10 mg/kg geeignet.

Allgemein können Mengen zwischen 0,1 mg bis zu 1000 mg, bezogen auf einen Erwachsenen von 75 kg Körpergewicht, appliziert werden.

### Liste der Abkürzungen:

- CH₂Cl₂: Dichlormethan
- DCI: Desorption-Chemical Ionisation
- DIP: Diisopropylether
- DMF: N,N-Dimethylformamid
- EE: Ethylacetat
- FAB: Fast Atom Bombardment
- h: Stunde(n)
- MeOH: Methanol
- Min: Minute(n)
- RT: Raumtemperatur
- TOTU: O-[Cyan(ethoxycarbonyl)methylenamino]-1,1,3,3-tetramethyluronium-tetrafluoroborat
- Zers.: Zersetzung

Die Erfindung wird durch die nachstehenden Beispiele erläutert.

### Beispiel 1

### 8-[3-(2-Aminoethoxy)-2,6-dichloro-benzyloxy]-2-methylchinolin

### a) 2,6-Dichloro-3-(2-phthaloyl-ethoxy)-toluol

5,0 g (28,2 mmol) 2,4-Dichloro-3-methyl-phenol und 9,2 g (28,2 mmol) Cs₂CO₃ in 100 ml DMSO wurden 10 min an RT gerührt. Es wurden 7,5 g (28,2 mmol) N-(2-Bromethyl)phthalimid zugefügt und die resultierende Reaktionsmischung 16 h an RT gerührt.
Es wurde mit H₂O versetzt und die Reaktionslösung mehrfach mit EE extrahiert. Die organischen Phasen wurden vereint, mit 1 N NaOH, 1 N HCl, H₂O und gesättigter NaCl-Lsg. gewaschen und über Na₂SO₄ getrocknet. Filtration, Abzug des Lösungsmittels und Chromatographie an Kieselgel mit EE/n-Heptan 1:4 lieferte 2,9 g der Titelverbindung.
Fp.: 148°C
R_{f} (SiO₂, EE/n-Heptan 1:4) = 0,15
MS (DCI): 350 (M+H)

### b) 2,6-Dichloro-3-(2-phthaloyl-ethoxy)-benzylbromid

Eine Lösung von 1,9 g (5,4 mmol) der Verbindung aus Beispiel 1a), 1,0 g (5,4 mmol) N-Bromsuccinimid und 50 mg Benzoylperoxid in 20 ml Chlorbenzol wurde 2 h am Rückfluß erhitzt. Es wurde zur Trockene eingeengt und der Rückstand in CH₂Cl₂ aufgenommen. Die erhaltene CH₂Cl₂-Lösung wurde mit ges. NaHCO₃-Lösung und H₂O gewaschen und über Na₂SO₄ getrocknet. Es wurde zur Trockene eingeengt und der Rückstand aus EE umkristallisiert. Absaugen und Trocknung der Kristalle im Hochvakuum lieferte 1,9 g der Titelverbindung.
Fp.: 171°C
R_{f} (SiO₂, EE/n-Heptan 1:2) = 0,19
MS (DCI) : 428/430 (M+H)

### c) 8-[2,6-Dichloro-3-(2-phthaloyl-ethoxy)-benzyloxy]-2-methylchinolin

Zu einer Suspension von 207,0 mg (4,3 mmol) einer 50 %igen Dispersion von NaH in Mineralöl in 30 ml abs. DMF wurden bei 0°C 700,0 mg (4,3 mmol) 8-Hydroxy-2-methylchinolin portionsweise hinzugegeben. Es wurde 30 min. bei 0°C gerührt und ebenfalls portionsweise 1,85 g (4,3 mmol) der Verbindung aus Beispiel 1b) zugefügt. Nach 1 h Rühren bei 0°C wurde die Reaktionslösung zur Trockene eingeengt, der Rückstand in wenig H₂O suspendiert und mehrfach mit CH₂Cl₂ extrahiert. Die vereinten organischen Extrakte wurden über Na₂SO₄ getrocknet, filtriert und eingeengt. Der erhaltene Rückstand wurde aus EE umkristallisiert. Absaugen und Trocknung der weißen Kristalle ergab 2,0 g der Titelverbindung.
Fp.: 176°C
R_{f} (SiO₂, EE/n-Heptan 1:2) = 0,13
MS (DCI) = 507 (M+M)

### d) 8-[3-(2-Aminoethoxy)-2,6-dichloro-benzyloxy]-2-methylchinolin

Eine Lösung von 1,8 g (3,5 mmol) der Verbindung aus Beispiel 1c) und 350 µl (7,2 mmol) Hydrazin-hydrat in 30 ml Ethanol wurde 1,5 h zum Rückfluß erhitzt. Es wurde zur Trockene eingeengt, der erhaltene Rückstand in Wasser suspendiert und die Suspension nach Zugabe von 2 N NaOH (pH ∼ 12) mehrfach mit CH₂Cl₂ extrahiert. Trocknung über Na₂SO₄, Abzug des Lösungsmittels und Reinigung durch Chromatographie an Kieselgel mit EE/MeOH/NH₄OH = 8:2:0,1 ergab 1,3 g der Titelverbindung.
Fp.: 123-125°C
R_{f} (EE/MeOH/NH₄OH 8:2:0,1) = 0,16
MS (DCI) = 377 (M+H)

### Beispiel 2

### 8-[3-(2-N-(trans-4-Aminocinnamoyl)amino-ethoxy)-2,6-dichlorobenzyloxy]-2-methylchinolin

### a) 8-[3-(2-(trans-4-(N-tert.-Butyloxycarbonyl)amino-cinnamoyl)amino-ethoxy)-benzyloxy]-2-methylchinolin

Eine Lösung von 120,0 mg (0,32 mmol) der Verbindung aus Beispiel 1a), 83,5 mg (0,32 mmol) trans-4-(N-tert.-Butyloxycarbonyl)-amino-zimtsäure, 56,0 µl N-Ethyl-diisopropylamin und 106,3 mg (0,32 mmol) TOTU in 5 ml abs. DMF wurden 3 h bei RT gerührt. Es wurde zur Trockene im Hochvakuum eingeengt, der Rückstand in CH₂Cl₂/Wasser aufgenommen und die organische Phase abgetrennt. Es wurde mit 10 %iger KHSO₄- und 10 % NaHCO₃-Lösung gewaschen und über Na₂SO₄ getrocknet. Filtration, Abzug des Lösungsmittels und chromatographische Reinigung an SiO₂ mit EE/Heptan 2:1 lieferte 130 mg der Titelverbindung.
Fp.: 116-118°C
R_{f} (EE/n-Heptan 4:1) = 0,27
MS (FAB): 622 (M+H)

### b) 8-[3-(2-N-(4-trans-Aminocinnamoyl)amino-ethoxy)-2,6-dichlorobenzyloxy]-2-methylchinolin

Eine Lösung von 65,0 mg (0,10 mmol) der Verbindung aus Beispiel 2a) und 260 µl Trifluoressigsäure in 5 ml CH₂Cl₂ wurde 5 h an RT gerührt. Es wurde zur Trockene eingeengt und der Rückstand mehrfach in Toluol aufgenommen und erneut zur Trockene eingeengt. Der verbleibende Rückstand wurde in wenig MeOH aufgenommen und die Titelverbindung durch Zugabe von Diisopropylether auskristallisiert. Es wurden 60 mg der Titelverbindung als Bistrifluoracetat isoliert.
Fp.: 158°C (Zers.)
R_{f} (EE/n-Heptan 10:1) = 0,18
MS (FAB): 522 (M+H)

### Beispiel 3

### 8-[2,6-Dichloro-3-((2-N-ethylaminocarbonyl)amino-ethoxy)-benzyloxy]-2-methylchinolin

80,0 mg (0,21 mmol) der Verbindung aus Beispiel 1d) und 33,0 µl (0,42 mmol) Ethylisocyanat in 2 ml CH₂Cl₂ wurden 1,5 h an RT gerührt. Es wurde zur Trockene eingeengt und der Rückstand mit wenig EE verrieben. Die Titelverbindung fiel als weißer Niederschlag an, der abgesaugt und mit wenig kaltem EE gewaschen wurde. Trocknung im Hochvakuum ergab 70 mg der gewünschten Verbindung.
Fp.: 153-154°C
R_{f} (EE/n-Heptan 1:2) = 0,49
MS (DCI): 448 (M+H)

### Beispiel 4

### 8-[2,6-Dichloro-3-(2-(3-phenylpropionyl)amino-ethoxy)-benzyloxy]-2-methylchinolin

Es wurden 90,0 mg (0,24 mmol) der Verbindung aus Beispiel 1d), 35,8 mg (0,24 mmol) 3-Phenylpropionsäure 42,0 µl (0,24 mmol) N-Ethyldiisopropylamin und 80,0 mg (0,24 mmol) TOTU in 5 ml abs. DMF nach dem in Beispiel 2a) angeführten Verfahren umgesetzt. Es resultierten nach Chromatographie an SiO₂ mit EE/n-Heptan 2:1 als Laufmittel 60,0 mg der Titelverbindung.
Fp.: 112-114°C
R_{f} (SiO₂, EE/n-Heptan 2:1) = 0,12
MS (DCI): 509 (M+H)

### Beispiel 5

### 8-[2,6-Dichloro-3-(trans-2-N-cinnamoylamino-ethoxy)-benzyloxy]-2-methylchinolin

100,0 mg (0,26 mmol) der Verbindung aus Beispiel 1d), 39,3 mg (0,26 mmol) trans-Zimtsäure, 46,6 µl (0,26 mmol) N-Ethyl-diisopropylamin und 88,8 mg (0,26 mmol) TOTU wurden in 5 ml abs. DMF nach dem in Beispiel 2a) angeführten Verfahren umgesetzt. Es resultieren nach Chromatographie an SiO₂ (EE/n-Heptan 2:1) 90 mg der Titelverbindung.
Fp.: 170-171°C
R_{f} (EE/n-Heptan 2:1) = 0,13
MS (DCI): 507 (M+H)

### Beispiel 6

### 8-[2,6-Dichloro-3-(2-N-(trans-4-methoxycinnamoyl)-amino-ethoxy)-benzyloxy]-2-methylchinolin

100,0 mg (0,26 mmol) der Verbindung aus Beispiel 1d), 47,2 mg (0,26 mmol) trans-p-Methoxyzimtsäure, 46,6 µl (0,26 mmol) N-Ethyl-diisopropylamin und 88,8 mg (0,26 mmol) TOTU wurden in 5 ml abs. DMF analog zu dem in Beispiel 2a) angeführten Verfahren umgesetzt. Es resultierten 69 mg der Titelverbindung.
Fp.: 182°C
R_{f} (EE/n-Heptan 2:1) = 0,12
MS (DCI): 537 (M+H)

### Beispiel 7

### 8-[2,6-Dichloro-3-((2-N-(trans-2-phenylcyclopropan-1-carbonyl)-amino-ethoxy)-benzyloxy]-2-methylchinolin

100,0 mg (0,26 mmol) der Verbindung aus Beispiel 1d), 43,0 mg (0,26 mmol) trans-2-Phenylcyclopropan-1-carbonsäure, 46 µl (0,26 mmol) N-Ethyldiisopropylamin und 88,8 mg (0,26 mmol) TOTU wurden entsprechend des in Beispiel 2a) angeführten Verfahrens umgesetzt. Man erhielt 65 mg der Titelverbindung.
Fp.: 108°C (Zers.)
R_{f} (SiO₂, EE/n-Heptan 2:1) = 0,15
MS (DCI): 521 (M+H)

### Beispiel 8

### 8-[2,6-Dichloro-3-(2-N-(trans-3-methoxycinnamoyl)-amino-ethoxy)-benzyloxy]-2-methylchinolin

100,0 mg (0,26 mmol) der Verbindung aus Beispiel 1d) wurden mit 47,2 mg (0,26 mmol) trans-m-Methoxyzimtsäure entsprechend dem in Beispiel 2a) angeführten Verfahren umgesetzt. Es resultierten 55,0 mg der Titelverbindung.
Fp.: 158-159°C
R_{f} (SiO₂, EE/n-Heptan 2:1) = 0,10
MS (DCI): 537 (M+H)

Die folgenden Beispiele 9-14 lassen sich analog zu den in den Beispielen 1 und 2 angeführten Verfahren unter Verwendung von N-(Brommethyl)-phthalimid, N-(3-Brompropyl)-phthalimid und N-(4-Brombutyl)-phthalimid anstelle des N-(2-Bromethyl)-phthalimids in Beispiel 1a) herstellen:

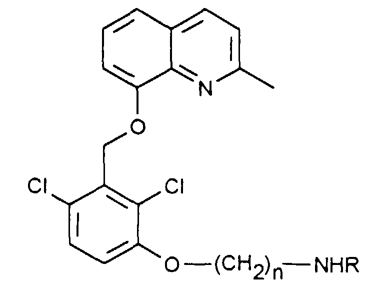

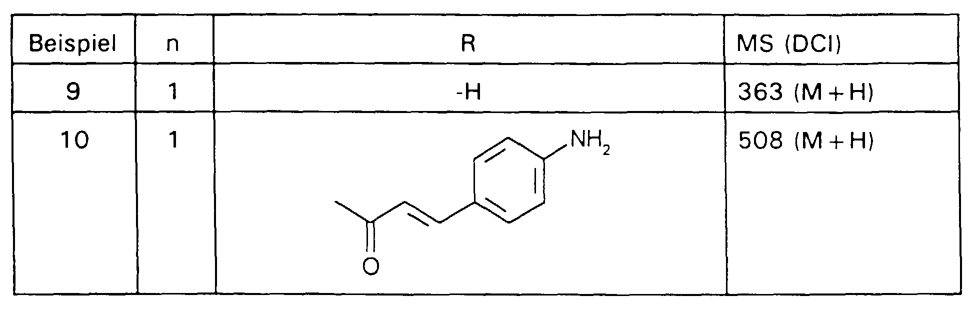

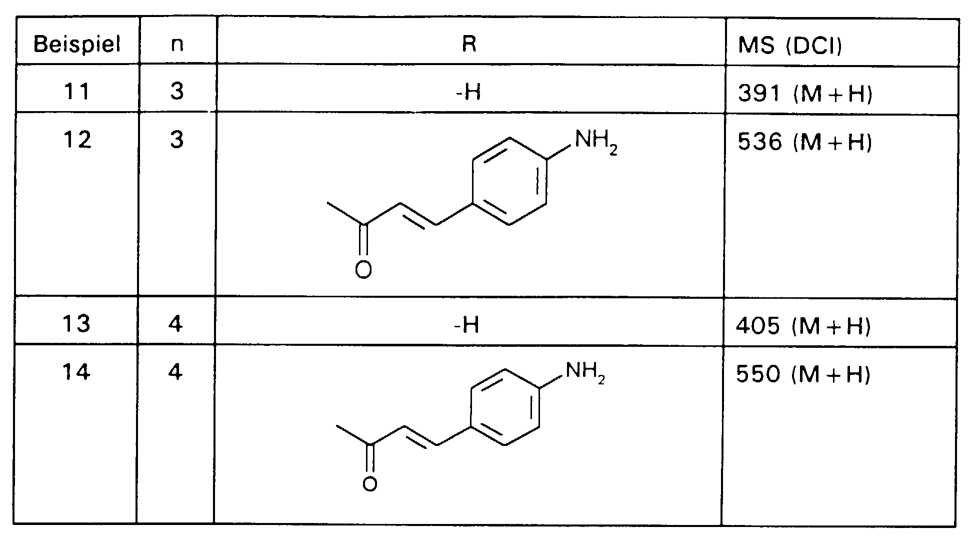

Die Beispiele 15-20 lassen sich analog zu den in den Beispielen 1 und 2 angeführten Verfahren unter Verwendung von 2,5-Dimethyl-8-hydroxychinolin, 2,7-Dimethyl-8-hydroxychinolin und 2,5,7-Trimethyl-8-hydroxychinolin - synthetisiert nach H. Fiedler, J. Prakt. Chemie, Bd. 13, 1961, 86 ff - anstelle des 8-Hydroxy-2-methylchinolins im Beispiel 1c) herstellen:

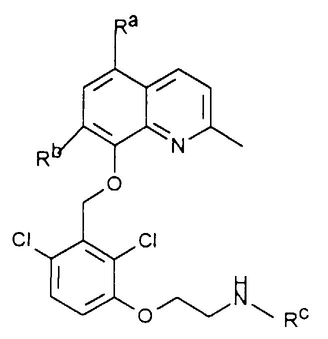

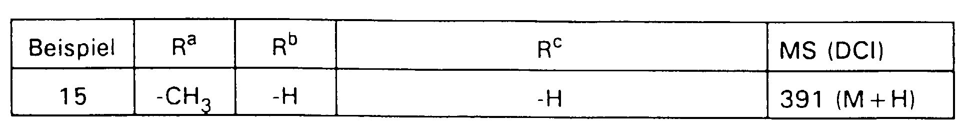

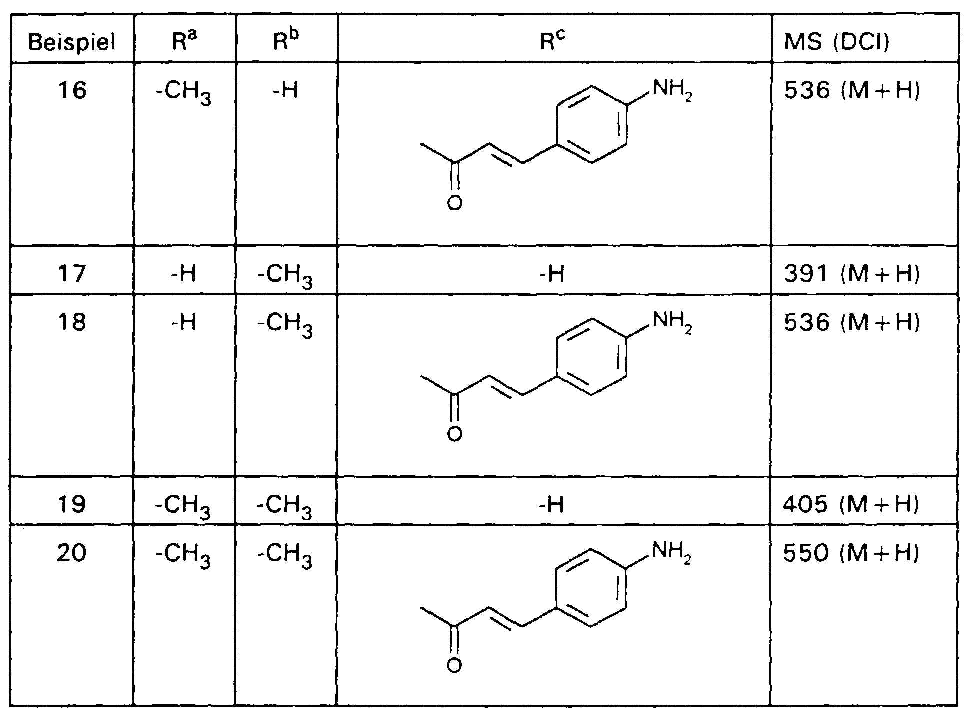

## Patentansprüche (Patentansprüche für folgende(n) Vertragsstaat(en): AT, BE, CH, DE, DK, ES, FR, GB, GR, IT, IE, LI, LU, NL, PT, SE)

1. Verbindungen der Formel (I), 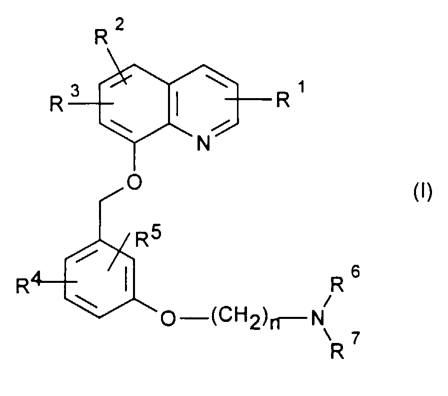 in welcher die Symbole die folgende Bedeutung haben:
R¹,R²,R³ gleich oder verschieden
1. (C₁-C₅)-Alkyl,
2. (C₆-C₁₀)-Aryl,
3. (C₁-C₃)-Alkyl-(C₆-C₁₀)-Aryl,
4. Halogen,
5. Wasserstoff,
6. (C₃-C₈)-Cycloalkyl,
7. CHO,
8. CO-O-(C₁-C₃)-alkyl,
9. COOH;
R⁴,R⁵ gleich oder verschieden
1. Wasserstoff,
2. Halogen,
3. (C₁-C₃)-Alkoxy,
4. Nitro,
5. Cyano,
6. S-(C₁-C₃)-Alkyl;
n eine Zahl von 1 bis 8;
R⁶
1. Wasserstoff,
2. (C₁-C₃)-Alkyl,
3. (C₃-C₅)-Alkylalkenyl,
4. (C₁-C₃)-Alkyl-(C₆-C₁₀)-Aryl;
R⁷ Wasserstoff und die folgenden substituierten oder unsubstituierten Acylreste:
(C₁-C₆)-Alkanoyl, (C₁-C₃)-Alkoxy-(C₂-C₆)-alkanoyl, (C₁-C₆)-Alkylcarbamoyl-(C₂-C₆)-alkanoyl, (C₆-C₁₂)-Aryl-(C₂-C₆)-alkanoyl, (C₃-C₇)-Alkenoyl, (C₃-C₈)-Cycloalkylcarbonyl, (C₅-C₇)-Cycloalkenylcarbonyl, (C₁-C₃)-Alkoxycarbonyl, (C₆-C₁₂)-Aryloxycarbonyl, (C₆-C₁₂)-Aroyl, (C₁-C₃)-Alkoxy-(C₆-C₁₂)-aroyl, Halogen-(C₆-C₁₂)-aroyl, (C₆-C₁₂)-Aryl-(C₃-C₆)-alkenoyl, (C₁-C₃)-Alkoxy-(C₆-C₁₂)-aryl-(C₃-C₆)-alkenoyl, (C₁-C₃)-Alkylendioxy-(C₆-C₁₂)-aryl-(C₃-C₆)-alkenoyl, Nitro-(C₆-C₁₂)-aryl-(C₃-C₆)-alkenoyl, Cyano-(C₆-C₁₂)-aryl-(C₃-C₆)-alkenoyl, Halogen-(C₆-C₁₂)-aryl-(C₃-C₆)-alkenoyl, Halogen-(C₁-C₃)-alkyl-(C₆-C₁₂)-aryl-(C₃-C₆)-alkenoyl, Hetero-(C₃-C₈)-cycloalkyl-(C₆-C₁₂)-aryl-(C₃-C₆)-alkenoyl, Amino-(C₆-C₁₂)-aryl-(C₃-C₆)-alkenoyl, (C₁-C₄)-Alkylamino-(C₆-C₁₂)-aryl-(C₃-C₆)-alkenoyl, (C₂-C₅)-Acylamino-(C₆-C₁₂)-arylcinnamoyl, (C₁-C₃)-Alkoxycarbonylamino-(C₆-C₁₂)-arylcinnamoyl, (C₁-C₄)-Alkylaminocarbonylaminocinnamoyl, Hetero-(C₆-C₁₂)-aryl-(C₂-C₆)-alkanoylamino-(C₆-C₁₂)-aryl-(C₃-C₆)-alkenoyl, (C₆-C₁₂)-Aroylamino-(C₆-C₁₂)-aryl-(C₃-C₆)-alkenoyl, Hetero-(C₆-C₁₂)-arylcarbonylamino-(C₆-C₁₂)-aryl-(C₃-C₆)-alkenoyl, (C₁-C₅)-Alkylsulfonylamino-(C₆-C₁₂)-aryl-(C₃-C₆)-alkenoyl, (C₁-C₅)-Alkylureido-(C₆-C₁₂)-aryl-(C₃-C₆)-alkenoyl, (C₂-C₆)-Alkanoyl-(C₆-C₁₂)-aryl-(C₃-C₆)-alkenoyl, (C₁-C₅)-Alkoxycarbonyl-(C₆-C₁₂)-aryl-(C₃-C₆)-alkenoyl, (C₁-C₅)-Alkylcarbamoyl-(C₆-C₁₂)-aryl-(C₃-C₆)-alkenoyl, (C₆-C₁₂)-Arylcarbamoyl-(C₆-C₁₂)-aryl-(C₃-C₆)-alkenoxyl, (C₆-C₁₂)-Aryl-(C₁-C₅)-alkoxycarbonyl, (C₁-C₅)-Alkylcarbamoyl, (C₆-C₁₂)-Arylcarbamoyl, (C₆-C₁₂)-Aroylcarbamoyl, (C₁-C₆)-Alkylsulfonyl, (C₆-C₁₂)-Arylsulfonyl, (C₆-C₁₂)-Aryl-(C₁-C₆)-alkylsulfonyl und Phthaloyl (für R⁶=R⁷),
sowie deren physiologisch verträgliche Salze, wobei die Verbindungen 8-[2,6-Dichlor-3-(2-phthalimidoethoxy)benzyloxy]-2-methylchinolin, 8-[3-(2-Aminoethoxy)-2,6-dichlorbenzyloxy]-2-methylchinolin und 8-[3-(2-Acetamidoethoxy)-2,6-dichlorbenzyloxy]-2-methylchinolin ausgenommen sind.

2. Verbindungen der Formel (I) gemäß Ansprüch 1, worin die Symbole folgende Bedeutung haben:
R¹,R²,R³ gleich oder verschieden
1. Wasserstoff,
2. (C₁-C₃)-Alkyl;
R⁴,R⁵ Halogen;
R⁶
1. Wasserstoff,
2. Methyl, Ethyl
3. Benzyl.

3. Verbindungen der Formel (I) gemäß Anspruch 1 oder 2, in der
R⁷
1. Wasserstoff oder
2. einen Acylrest der gemäß Anspruch 1 angegebene Bedeutung darstellt.

4. Verbindungen der Formel (I) gemäß den Ansprüchen 1 bis 3, in der die Symbole folgende Bedeutungen haben:
R¹,R²,R³ gleich oder verschieden
1. Wasserstoff,
2. Methyl, Ethyl, Propyl;
R⁴,R⁵ Chlor;
n 1 bis 4;
R⁶ Wasserstoff;
R⁷
1. Wasserstoff,
2. (C₂-C₅)-Alkanoyl,
3. (C₃-C₅)-Alkenoyl,
4. (C₁-C₅)-Alkylaminocarbonyl,
5. (C₆-C₁₀)-Aryl-(C₁-C₃)-alkylaminocarbonyl,
6. (C₁-C₅)-Alkyloxycarbonyl,
7. (C₆-C₁₀)-Aryl-(C₁-C₃)-alkyloxycarbonyl,
8. (C₆-C₁₀)-Aryl-(C₃-C₇)-cycloalkylcarbonyl,
9. ein trans-Zimtsäurerest, dessen Phenylring substituiert ist mit bis zu 2 gleichen oder verschiedenen Resten aus der Reihe
a) Wasserstoff,
b) (C₁-C₃)-Alkyl,
c) Amino,
d) (C₁-C₃)-Mono- und -Dialkylamino,
e) Halogen,
f) (C₁-C₃)-Halogenalkyl,
g) (C₂-C₅)-Acylamino und
h) (C₁-C₃)-Alkoxy.

5. Verfahren zur Herstellung von Verbindungen der Formel (I) gemäß den Ansprüchen 1 bis 4, dadurch gekennzeichnet, daß man
a) eine Verbindung der Formel (II) 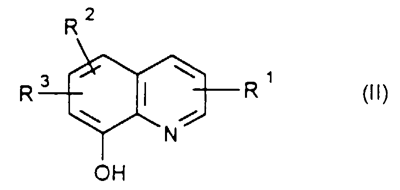 worin R¹, R² und R³ wie oben definiert sind, mit einer Verbindung der Formel (III) 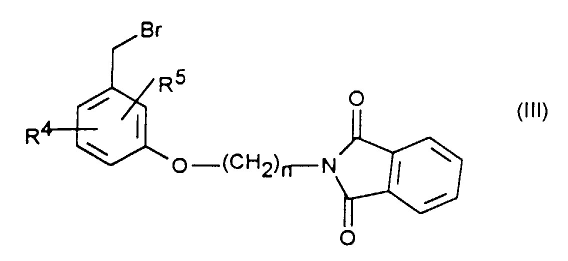 worin R⁴, R⁵ und n wie oben definiert sind, in Gegenwart von Metallhydriden, wie Lithium-, Kalium- oder Natriumhydrid, oder Alkalimetallcarbonaten, wie Natrium-, Kalium- oder Cäsiumcarbonat, in einem inerten Lösungsmittel, wie DMF oder DMSO, bei Temperaturen von 0°C bis 60 °C zu einer Verbindung der Formel (IV) 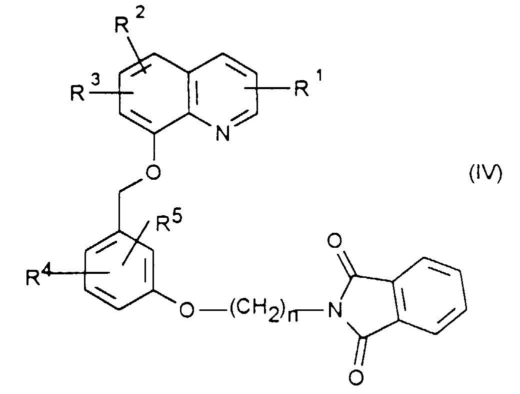 wobei die Symbole und Variablen wie oben definiert sind, umsetzt;
b) eine Verbindung der Formel (IV) durch Hydrazinolyse in Ethanol bei Rückfluß in eine Verbindung der Formel (Ia), 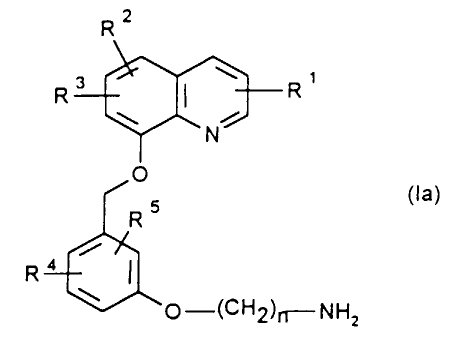 worin R¹, R², R³, R⁴, R⁵ und n wie oben definiert sind, überführt;
c) gegebenenfalls die Verbindungen der Formel (Ia) nach bekannten Methoden acyliert und/oder alkyliert, und
d) gegebenenfalls die erhaltenenen Verbindungen der Formel (I) nach bekannten Methoden in ihre physiologisch verträglichen Salze überführt.

6. Verwendung der Verbindungen der Formel (I) gemäß den Ansprüchen 1 bis 4 als Heilmittel.

7. Heilmittel enthaltend mindestens eine Verbindung der Formel (I) gemäß den Ansprüchen 1 bis 4.

## Patentansprüche (Patentansprüche für folgende(n) Vertragsstaat(en): FI)

1. Verbindungen der Formel (I), 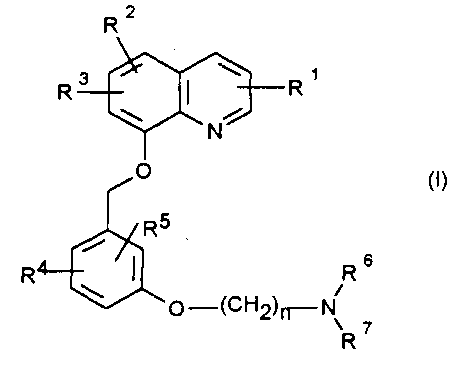 in welcher die Symbole die folgende Bedeutung haben:
R¹,R²,R³ gleich oder verschieden
1. (C₁-C₅)-Alkyl,
2. (C₆-C₁₀)-Aryl,
3. (C₁-C₃)-Alkyl-(C₆-C₁₀)-Aryl,
4. Halogen,
5. Wasserstoff,
6. (C₃-C₈)-Cycloalkyl,
7. CHO,
8. CO-O-(C₁-C₃)-alkyl,
9. COOH;
R⁴,R⁵ gleich oder verschieden
1. Wasserstoff,
2. Halogen,
3. (C₁-C₃)-Alkoxy,
4. Nitro,
5. Cyano,
6. S-(C₁-C₃)-Alkyl;
n eine Zahl von 1 bis 8;
R⁶
1. Wasserstoff,
2. (C₁-C₃)-Alkyl,
3. (C₃-C₅)-Alkylalkenyl,
4. (C₁-C₃)-Alkyl-(C₆-C₁₀)-Aryl;
R⁷ Wasserstoff und die folgenden substituierten oder unsubstituierten Acylreste:
(C₁-C₆)-Alkanoyl, (C₁-C₃)-Alkoxy-(C₂-C₆)-alkanoyl, (C₁-C₆)-Alkylcarbamoyl-(C₂-C₆)-alkanoyl, (C₆-C₁₂)-Aryl-(C₂-C₆)-alkanoyl, (C₃-C₇)-Alkenoyl, (C₃-C₈)-Cycloalkylcarbonyl, (C₅-C₇)-Cycloalkenylcarbonyl, (C₁-C₃)-Alkoxycarbonyl, (C₆-C₁₂)-Aryloxycarbonyl, (C₆-C₁₂)-Aroyl, (C₁-C₃)-Alkoxy-(C₆-C₁₂)-aroyl, Halogen-(C₆-C₁₂)-aroyl, (C₆-C₁₂)-Aryl-(C₃-C₆)-alkenoyl, (C₁-C₃)-Alkoxy-(C₆-C₁₂)-aryl-(C₃-C₆)-alkenoyl, (C₁-C₃)-Alkylendioxy-(C₆-C₁₂)-aryl-(C₃-C₆)-alkenoyl, Nitro-(C₆-C₁₂)-aryl-(C₃-C₆)-alkenoyl, Cyano-(C₆-C₁₂)-aryl-(C₃-C₆)-alkenoyl, Halogen-(C₆-C₁₂)-aryl-(C₃-C₆)-alkenoyl, Halogen-(C₁-C₃)-alkyl-(C₆-C₁₂)-aryl-(C₃-C₆)-alkenoyl, Hetero-(C₃-C₈)-cycloalkyl-(C₆-C₁₂)-aryl-(C₃-C₆)-alkenoyl, Amino-(C₆-C₁₂)-aryl-(C₃-C₆)-alkenoyl, (C₁-C₄)-Alkylamino-(C₆-C₁₂)-aryl-(C₃-C₆)-alkenoyl, (C₂-C₅)-Acylamino-(C₆-C₁₂)-arylcinnamoyl, (C₁-C₃)-Alkoxycarbonylamino-(C₆-C₁₂)-arylcinnamoyl, (C₁-C₄)-Alkylaminocarbonylaminocinnamoyl, Hetero-(C₆-C₁₂)-aryl-(C₂-C₆)-alkanoylamino-(C₆-C₁₂)-aryl-(C₃-C₆)-alkenoyl, (C₆-C₁₂)-Aroylamino-(C₆-C₁₂)-aryl-(C₃-C₆)-alkenoyl, Hetero-(C₆-C₁₂)-arylcarbonylamino-(C₆-C₁₂)-aryl-(C₃-C₆)-alkenoyl, (C₁-C₅)-Alkylsulfonylamino-(C₆-C₁₂)-aryl-(C₃-C₆)-alkenoyl, (C₁-C₅)-Alkylureido-(C₆-C₁₂)-aryl-(C₃-C₆)-alkenoyl, (C₂-C₆)-Alkanoyl-(C₆-C₁₂)-aryl-(C₃-C₆)-alkenoyl, (C₁-C₅)-Alkoxycarbonyl-(C₆-C₁₂)-aryl-(C₃-C₆)-alkenoyl, (C₁-C₅)-Alkylcarbamoyl-(C₆-C₁₂)-aryl-(C₃-C₆)-alkenoyl, (C₆-C₁₂)-Arylcarbamoyl-(C₆-C₁₂)-aryl-(C₃-C₆)-alkenoxyl, (C₆-C₁₂)-Aryl-(C₁-C₅)-alkoxycarbonyl, (C₁-C₅)-Alkylcarbamoyl, (C₆-C₁₂)-Arylcarbamoyl, (C₆-C₁₂)-Aroylcarbamoyl, (C₁-C₆)-Alkylsulfonyl, (C₆-C₁₂)-Arylsulfonyl, (C₆-C₁₂)-Aryl-(C₁-C₆)-alkylsulfonyl und Phthaloyl (für R⁶ = R⁷),
sowie deren physiologisch verträgliche Salze.

2. Verbindungen der Formel (I) gemäß Ansprüch 1, worin die Symbole folgende Bedeutung haben:
R¹,R²,R³ gleich oder verschieden
1. Wasserstoff,
2. (C₁-C₃)-Alkyl;
R⁴,R⁵ Halogen;
R⁶
1. Wasserstoff,
2. Methyl, Ethyl
3. Benzyl.

3. Verbindungen der Formel (I) gemäß Anspruch 1 oder 2, in der
R⁷
1. Wasserstoff oder
2. einen Acylrest der gemäß Anspruch 1 angegebene Bedeutung darstellt.

4. Verbindungen der Formel (I) gemäß den Ansprüchen 1 bis 3, in der die Symbole folgende Bedeutungen haben:
R¹,R²,R³ gleich oder verschieden
1. Wasserstoff,
2. Methyl, Ethyl, Propyl;
R⁴,R⁵ Chlor;
n 1 bis 4;
R⁶ Wasserstoff;
R⁷
1. Wasserstoff,
2. (C₂-C₅)-Alkanoyl,
3. (C₃-C₅)-Alkenoyl,
4. (C₁-C₅)-Alkylaminocarbonyl,
5. (C₆-C₁₀)-Aryl-(C₁-C₃)-alkylaminocarbonyl,
6. (C₁-C₅)-Alkyloxycarbonyl,
7. (C₆-C₁₀)-Aryl-(C₁-C₃)-alkyloxycarbonyl,
8. (C₆-C₁₀)-Aryl-(C₃-C₇)-cycloalkylcarbonyl,
9. ein trans-Zimtsäurerest, dessen Phenylring substituiert ist mit bis zu 2 gleichen oder verschiedenen Resten aus der Reihe
a) Wasserstoff,
b) (C₁-C₃)-Alkyl,
c) Amino,
d) (C₁-C₃)-Mono- und -Dialkylamino,
e) Halogen,
f) (C₁-C₃)-Halogenalkyl,
g) (C₂-C₅)-Acylamino und
h) (C₁-C₃)-Alkoxy.

5. Verfahren zur Herstellung von Verbindungen der Formel (I) gemäß den Ansprüchen 1 bis 4, dadurch gekennzeichnet, daß man
a) eine Verbindung der Formel (II) 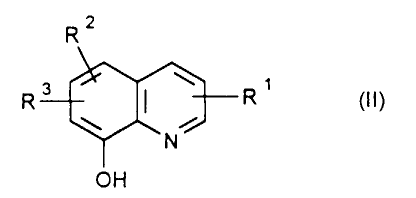 worin R¹, R² und R³ wie oben definiert sind, mit einer Verbindung der Formel (III) 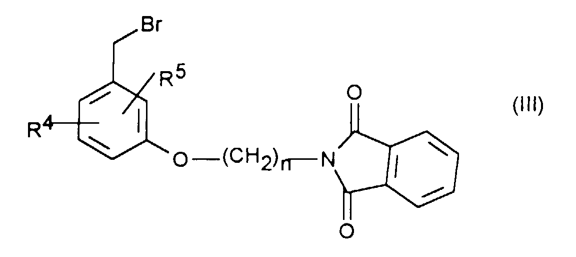 worin R⁴, R⁵ und n wie oben definiert sind, in Gegenwart von Metallhydriden, wie Lithium-, Kalium- oder Natriumhydrid, oder Alkalimetallcarbonaten, wie Natrium-, Kalium- oder Cäsiumcarbonat, in einem inerten Lösungsmittel, wie DMF oder DMSO, bei Temperaturen von 0°C bis 60 °C zu einer Verbindung der Formel (IV) 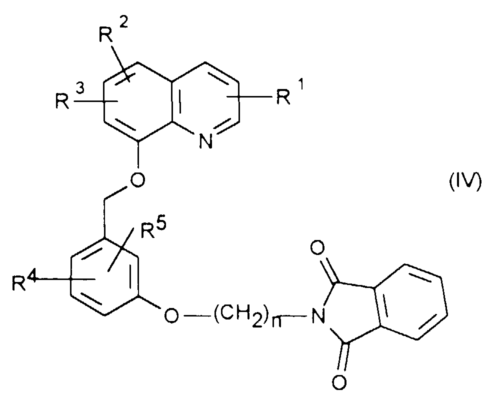 wobei die Symbole und Variablen wie oben definiert sind, umsetzt;
b) eine Verbindung der Formel (IV) durch Hydrazinolyse in Ethanol bei Rückfluß in eine Verbindung der Formel (Ia), 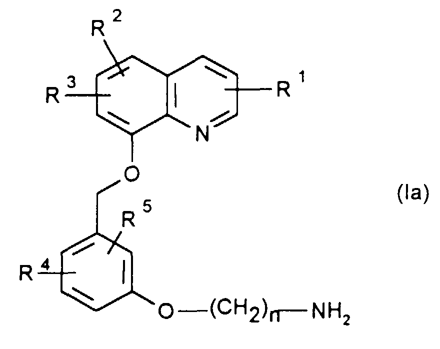 worin R¹, R², R³, R⁴, R⁵ und n wie oben definiert sind, überführt;
c) gegebenenfalls die Verbindungen der Formel (Ia) nach bekannten Methoden acyliert und/oder alkyliert, und
d) gegebenenfalls die erhaltenenen Verbindungen der Formel (I) nach bekannten Methoden in ihre physiologisch verträglichen Salze überführt.

6. Verwendung der Verbindungen der Formel (I) gemäß den Ansprüchen 1 bis 4 als Heilmittel.

7. Heilmittel enthaltend mindestens eine Verbindung der Formel (I) gemäß den Ansprüchen 1 bis 4.

## Claims (Claims for the following Contracting State(s): AT, BE, CH, DE, DK, ES, FR, GB, GR, IT, IE, LI, LU, NL, PT, SE)

1. A compound of the formula (I), 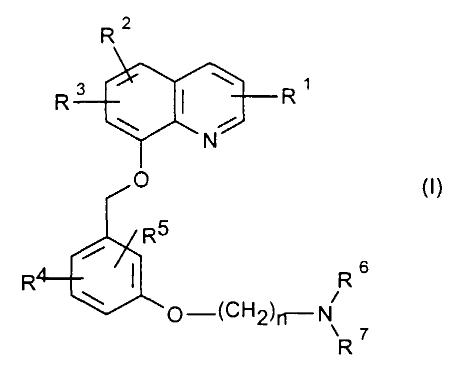 in which the symbols have the following meaning:
R¹,R²,R³ identical or different
1. (C₁-C₅)-alkyl,
2. (C₆-C₁₀)-aryl,
3. (C₁-C₃)-alkyl-(C₆-C₁₀)-aryl,
4. halogen,
5. hydrogen,
6. (C₃-C₈)-cycloalkyl,
7. CHO,
8. CO-O-(C₁-C₃)-alkyl,
9. COOH;
R⁴,R⁵ identical or different
1. hydrogen,
2. halogen,
3. (C₁-C₃)-alkoxy,
4. nitro,
5. cyano,
6. S-(C₁-C₃)-alkyl;
n a number from 1 to 8;
R⁶
1. hydrogen,
2. (C₁-C₃)-alkyl,
3. (C₃-C₅)-alkylalkenyl,
4. (C₁-C₃)-alkyl-(C₆-C₁₀)-aryl;
R⁷ hydrogen and the following substituted or unsubstituted acyl radicals:
(C₁-C₆)alkanoyl, (C₁-C₃)-alkoxy-(C₂-C₆)-alkanoyl, (C₁-C₆)-alkylcarbamoyl-(C₂-C₆)-alkanoyl, (C₆-C₁₂)-aryl-(C₂-C₆)-alkanoyl, (C₃-C₇)-alkenoyl, (C₃-C₈)-cycloalkylcarbonyl, (C₅-C₇)-cycloalkenylcarbonyl, (C₁-C₃)-alkoxycarbonyl, (C₆-C₁₂)-aryloxycarbonyl, (C₆-C₁₂)-aroyl, (C₁-C₃)-alkoxy-(C₆-C₁₂)-aroyl, halogen-(C₆-C₁₂)-aroyl, (C₆-C₁₂)-aryl-(C₃-C₆)-alkenoyl, (C₁-C₃)-alkoxy-(C₆-C₁₂)-aryl-(C₃-C₆)-alkenoyl, (C₁-C₃)-alkylenedioxy-(C₆-C₁₂)-aryl-(C₃-C₆)-alkenoyl, nitro-(C₆-C₁₂)-aryl-(C₃-C₆)-alkenoyl, cyano-(C₆-C₁₂)-aryl-(C₃-C₆)-alkenoyl, halo-(C₆-C₁₂)-aryl-(C₃-C₆)-alkenoyl, halo-(C₁-C₃)-alkyl-(C₆-C₁₂)-aryl-(C₃-C₆)-alkenoyl, hetero-(C₃-C₈)-cycloalkyl-(C₆-C₁₂)-aryl-(C₃-C₆)-alkenoyl, amino(C₆-C₁₂)-aryl-(C₃-C₆)-alkenoyl, (C₁-C₄)-alkylamino-(C₆-C₁₂)-aryl-(C₃-C₆)-alkenoyl, (C₂-C₅)-acylamino-(C₆-C₁₂)-arylcinnamoyl, (C₁-C₃)alkoxycarbonylamino-(C₆-C₁₂)-arylcinnamoyl, (C₁-C₄)-alkylaminocarbonylaminocinnamoyl, hetero-(C₆-C₁₂)-aryl-(C₂-C₆)-alkanoylamino-(C₆-C₁₂)-aryl-(C₃-C₆)-alkenoyl, (C₆-C₁₂) aroylamino-(C₆-C₁₂)aryl-(C₃-C₆)-alkenoyl, hetero-(C₆-C₁₂)-arylcarbonylamino-(C₆-C₁₂)-aryl-(C₃-C₆)-alkenoyl, (C₁-C₅)-alkylsulfonylamino-(C₆-C₁₂)-aryl-(C₃-C₆)-alkenoyl, (C₁-C₅)-alkylureido-(C₆-C₁₂)-aryl-(C₃-C₆)-alkenoyl, (C₂-C₆)-alkanoyl-(C₆-C₁₂)-aryl-(C₃-C₆)-alkenoyl, (C₁-C₅)-alkoxycarbonyl-(C₆-C₁₂)-aryl-(C₃-C₆)-alkenoyl, (C₁-C₅)-alkylcarbamoyl-(C₆-C₁₂)-aryl-(C₃-C₆)-alkenoyl, (C₆-C₁₂)-arylcarbamoyl-(C₆-C₁₂)-aryl-(C₃-C₆)-alkenoxyl, (C₆-C₁₂)-aryl-(C₁-C₅)-alkoxycarbonyl, (C₁-C₅)-alkylcarbamoyl, (C₆-C₁₂)-arylcarbamoyl, (C₆-C₁₂)-aroylcarbamoyl, (C₁-C₆)-alkylsulfonyl, (C₆-C₁₂)-arylsulfonyl, (C₆-C₁₂)-aryl-(C₁-C₆)-alkylsulfonyl and phthaloyl (for R⁶=R⁷),
and its physiologically tolerated salts, the compounds 8-[2,6-dichloro-3-(2-phthalimidoethoxy)benzyloxy]-2-methylquinoline, 8-[3-(2-aminoethoxy)-2,6-dichlorobenzyloxy]-2-methylquinoline and 8-[3-(2-acetamidoethoxy)-2,6-dichlorobenzyloxy]-2-methylquinoline being excluded.

2. A compound of the formula (I) as claimed in claim 1, in which the symbols have the following meaning:
R¹,R²,R³ identical or different
1. hydrogen,
2. (C₁-C₃)-alkyl;
R⁴,R⁵ halogen;
R⁶
1. hydrogen,
2. methyl, ethyl
3. benzyl.

3. A compound of the formula (I) as claimed in claim 1 or 2, in which
R⁷ is
1. hydrogen or
2. an acyl radical as defined in claim 1.

4. A compound of the formula (I) as claimed in claims 1 to 3, in which the symbols have the following meanings:
R¹,R²,R³ identical or different
1. hydrogen,
2. methyl, ethyl, propyl;
R⁴,R⁵ chlorine;
n 1 to 4;
R⁶ hydrogen;
R⁷
1. hydrogen,
2. (C₂-C₅)-alkanoyl,
3. (C₃-C₅)-alkenoyl,
4. (C₁-C₅)-alkylaminocarbonyl,
5. (C₆-C₁₀)-aryl-(C₁-C₃)-alkylaminocarbonyl,
6. (C₁-C₅)-alkyloxycarbonyl,
7. (C₆-C₁₀)-aryl-(C₁-C₃)-alkyloxycarbonyl,
8. (C₆-C₁₀)-aryl-(C₃-C₇)-cycloalkylcarbonyl,
9. a trans-cinnamic acid residue whose phenyl ring is substituted by up to 2 identical or different radicals from the series
a) hydrogen,
b) (C₁-C₃)-alkyl,
c) amino,
d) (C₁-C₃)-mono- and -dialkylamino,
e) halogen,
f) (C₁-C₃)haloalkyl,
g) (C₂-C₅)acylamino and
h) (C₁-C₃)-alkoxy.

5. A process for preparing compounds of the formula (I) as claimed in claims 1 to 4, which comprises
a) reacting a compound of the formula (II) 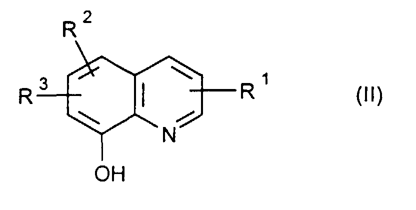 in which R¹, R² and R³ are as defined above, with a compound of the formula (III) 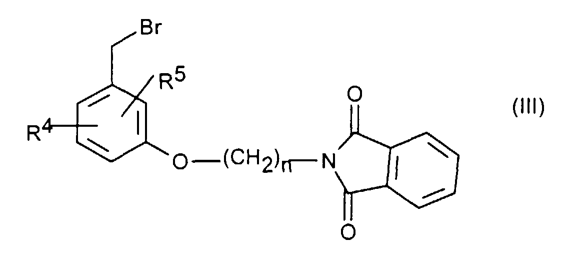 in which R⁴, R⁵ and n are as defined above, in the presence of metal hydrides such as lithium, potassium or sodium hydride, or alkali metal carbonates such as sodium, potassium or cesium carbonate, in an inert solvent such as DMF or DMSO, at temperatures from 0°C to 60°C to give a compound of the formula (IV) 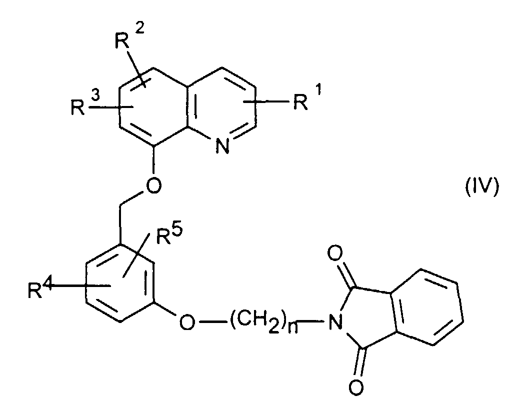 where the symbols and variables are as defined above;
b) converting a compound of the formula (IV) by hydrazinolysis in ethanol under reflux into a compound of the formula (la), 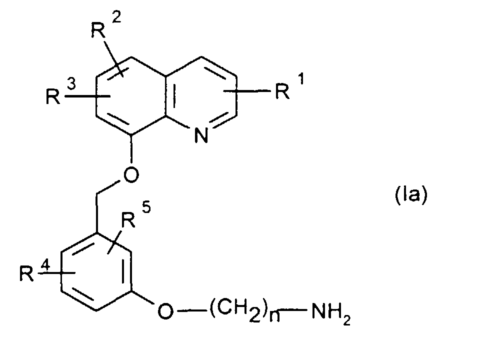 in which R¹, R², R³, R⁴, R⁵ and n are as defined above;
c) where appropriate acylating and/or alkylating the compounds of the formula (la) by known methods, and
d) where appropriate converting the resulting compounds of the formula (I) into their physiologically tolerated salts by known methods.

6. The use of the compounds of the formula (I) as claimed in claims 1 to 4 as medicine.

7. A medicine containing at least one compound of the formula (I) as claimed in claims 1 to 4.

## Claims (Claims for the following Contracting State(s): FI)

1. A compound of the formula (I), 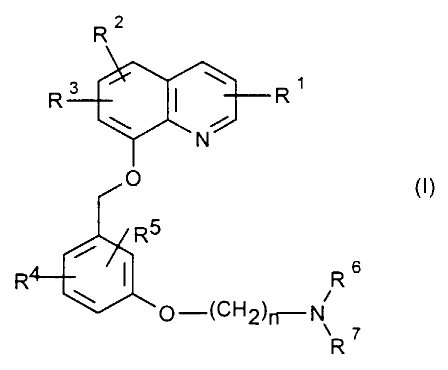 in which the symbols have the following meaning:
R¹,R²,R³ identical or different
1. (C₁-C₅)-alkyl,
2. (C₆-C₁₀)-aryl,
3. (C₁-C₃)-alkyl-(C₆-C₁₀)-aryl,
4. halogen,
5. hydrogen,
6. (C₃-C₈)-cycloalkyl,
7. CHO,
8. CO-O-(C₁-C₃)-alkyl,
9. COOH;
R⁴,R⁵ identical or different
1. hydrogen,
2. halogen,
3. (C₁-C₃)-alkoxy,
4. nitro,
5. cyano,
6. S-(C₁-C₃)-alkyl;
n a number from 1 to 8;
R⁶
1. hydrogen,
2. (C₁-C₃)-alkyl,
3. (C₃-C₅)-alkylalkenyl,
4. (C₁-C₃)-alkyl-(C₆-C₁₀)-aryl;
R⁷ hydrogen and the following substituted or unsubstituted acyl radicals:
(C₁-C₆)-alkanoyl, (C₁-C₃)-alkoxy-(C₂-C₆)-alkanoyl, (C₁-C₆)-alkylcarbamoyl-(C₂-C₆)-alkanoyl, (C₆-C₁₂)-aryl-(C₂-C₆)-alkanoyl, (C₃-C₇)-alkenoyl, (C₃-C₈)-cycloalkylcarbonyl, (C₅-C₇)-cycloalkenylcarbonyl, (C₁-C₃)-alkoxycarbonyl, (C₆-C₁₂)-aryloxycarbonyl, (C₆-C₁₂)-aroyl, (C₁-C₃)-alkoxy-(C₆-C₁₂)-aroyl, halogen-(C₆-C₁₂)-aroyl, (C₆-C₁₂)-aryl-(C₃-C₆)-alkenoyl, (C₁-C₃)-alkoxy-(C₆-C₁₂)-aryl-(C₃-C₆)-alkenoyl, (C₁-C₃)-alkylenedioxy-(C₆-C₁₂)-aryl-(C₃-C₆)-alkenoyl, nitro-(C₆-C₁₂)-aryl-(C₃-C₆)-alkenoyl, cyano-(C₆-C₁₂)-aryl-(C₃-C₆)-alkenoyl, halo-(C₆-C₁₂)-aryl-(C₃-C₆)-alkenoyl, halo-(C₁-C₃)-alkyl-(C₆-C₁₂)-aryl-(C₃-C₆)-alkenoyl, hetero-(C₃-C₈)-cycloalkyl-(C₆-C₁₂)-aryl-(C₃-C₆)-alkenoyl, amino(C₆-C₁₂)-aryl-(C₃-C₆)-alkenoyl, (C₁-C₄)-alkylamino-(C₆-C₁₂)-aryl-(C₃-C₆)-alkenoyl, (C₂-C₅)-acylamino-(C₆-C₁₂)-arylcinnamoyl, (C₁-C₃)-alkoxycarbonylamino-(C₆-C₁₂)-arylcinnamoyl, (C₁-C₄)-alkylaminocarbonylaminocinnamoyl, hetero-(C₆-C₁₂)-aryl(C₂-C₆)-alkanoylamino-(C₆-C₁₂)-aryl-(C₃-C₆)-alkenoyl, (C₆-C₁₂)-aroylamino-(C₆-C₁₂)-aryl-(C₃-C₆)alkenoyl, hetero-(C₆-C₁₂)-arylcarbonylamino-(C₆-C₁₂)-aryl-(C₃-C₆)-alkenoyl, (C₁-C₅)-alkylsulfonylamino-(C₆-C₁₂)-aryl-(C₃-C₆)-alkenoyl, (C₁-C₅)-alkylureido-(C₆-C₁₂)-aryl-(C₃-C₆)-alkenoyl, (C₂-C₆)-alkanoyl-(C₆-C₁₂)-aryl-(C₃-C₆)-alkenoyl, (C₁-C₅)-alkoxycarbonyl-(C₆-C₁₂)-aryl-(C₃-C₆)-alkenoyl, (C₁-C₅)-alkylcarbamoyl-(C₆-C₁₂)-aryl-(C₃-C₆)-alkenoyl, (C₆-C₁₂)-arylcarbamoyl-(C₆-C₁₂)-aryl-(C₃-C₆)-alkenoxyl, (C₆-C₁₂)-aryl-(C₁-C₅)-alkoxycarbonyl, (C₁-C₅)-alkylcarbamoyl, (C₆-C₁₂)-arylcarbamoyl, (C₆-C₁₂)-aroylcarbamoyl, (C₁-C₆)-alkylsulfonyl, (C₆-C₁₂)-arylsulfonyl, (C₆-C₁₂)-aryl-(C₁-C₆)-alkylsulfonyl and phthaloyl (for R⁶=R⁷),
and its physiologically tolerated salts.

2. A compound of the formula (I) as claimed in claim 1, in which the symbols have the following meaning:
R¹,R²,R³ identical or different
1. hydrogen,
2. (C₁-C₃)-alkyl;
R⁴,R⁵ halogen;
R⁶
1. hydrogen,
2. methyl, ethyl
3. benzyl.

3. A compound of the formula (I) as claimed in claim 1 or 2, in which
R⁷ is
1. hydrogen or
2. an acyl radical as defined in claim 1.

4. A compound of the formula (I) as claimed in claims 1 to 3, in which the symbols have the following meanings:
R¹,R²,R³ identical or different
1. hydrogen,
2. methyl, ethyl, propyl;
R⁴,R⁵ chlorine;
n 1 to 4;
R⁶ hydrogen;
R⁷
1. hydrogen,
2. (C₂-C₅)-alkanoyl,
3. (C₃-C₅)-alkenoyl,
4. (C₁-C₅)-alkylaminocarbonyl,
5. (C₆-C₁₀)-aryl-(C₁-C₃)-alkylaminocarbonyl,
6. (C₁-C₅)-alkyloxycarbonyl,
7. (C₆-C₁₀)-aryl-(C₁-C₃)-alkyloxycarbonyl,
8. (C₆-C₁₀)-aryl-(C₃-C₇)-cycloalkylcarbonyl,
9. a trans-cinnamic acid residue whose phenyl ring is substituted by up to 2 identical or different radicals from the series
a) hydrogen,
b) (C₁-C₃)-alkyl,
c) amino,
d) (C₁-C₃)-mono- and -dialkylamino,
e) halogen,
f) (C₁-C₃)-haloalkyl,
g) (C₂-C₅)-acylamino and
h) (C₁-C₃)-alkoxy.

5. A process for preparing compounds of the formula (I) as claimed in claims 1 to 4, which comprises
a) reacting a compound of the formula (II) 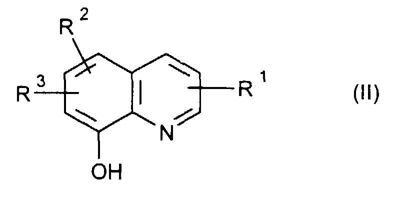 in which R¹, R² and R³ are as defined above, with a compound of the formula (III) 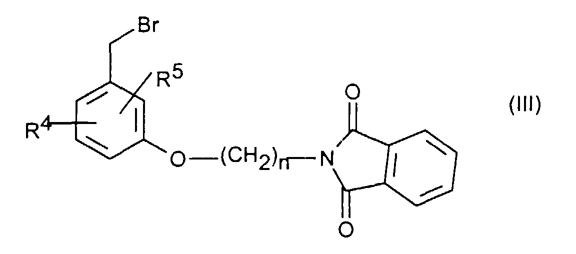 in which R⁴, R⁵ and n are as defined above, in the presence of metal hydrides such as lithium, potassium or sodium hydride, or alkali metal carbonates such as sodium, potassium or cesium carbonate, in an inert solvent such as DMF or DMSO, at temperatures from 0°C to 60°C to give a compound of the formula (IV) 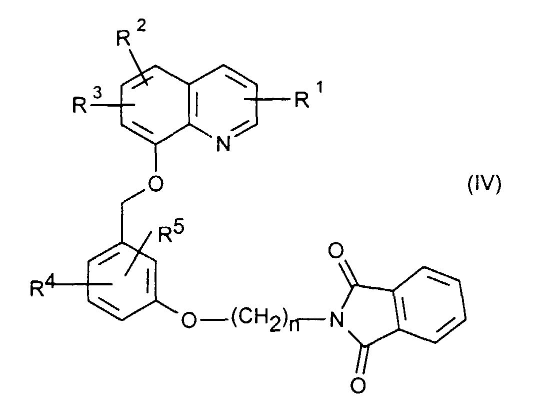 where the symbols and variables are as defined above;
b) converting a compound of the formula (IV) by hydrazinolysis in ethanol under reflux into a compound of the formula (la), 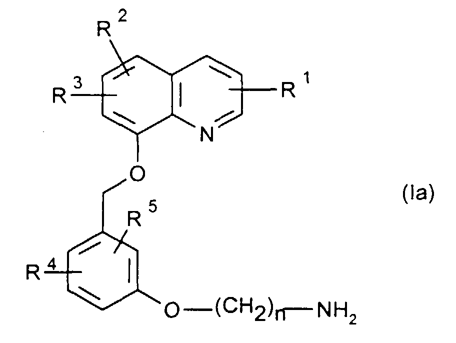 in which R¹, R², R³, R⁴, R⁵ and n are as defined above;
c) where appropriate acylating and/or alkylating the compounds of the formula (la) by known methods, and
d) where appropriate converting the resulting compounds of the formula (I) into their physiologically tolerated salts by known methods.

6. The use of the compounds of the formula (I) as claimed in claims 1 to 4 as medicine.

7. A medicine containing at least one compound of the formula (I) as claimed in claims 1 to 4.

## Revendications (Revendications pour l'(les) Etat(s) contractant(s) suivant(s): AT, BE, CH, DE, DK, ES, FR, GB, GR, IT, IE, LI, LU, NL, PT, SE)

1. Composés de formule (I) 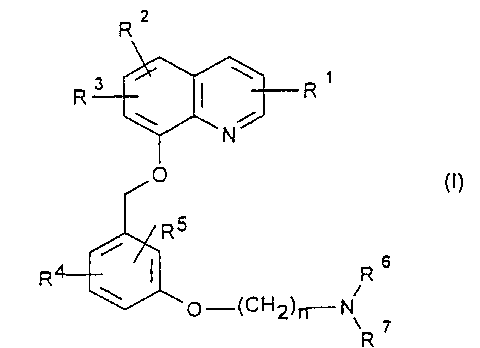 dans laquelle les symboles ont les significations suivantes:
R¹, R², R³ sont identiques ou différents et représentent
1. un groupe alkyle en C₁-C₅,
2. un groupe aryle en C₆-C₁₀,
3. un groupe alkyl(C₁-C₃)-aryle(C₆-C₁₀),
4. un atome d'halogène,
5. un atome d'hydrogène,
6. un groupe cycloalkyle en C₃-C₈,
7. CHO,
8. un groupe CO-O-alkyle(C₁-C₃),
9. COOH;
R⁴, R⁵ sont identiques ou différents et représentent
1. un atome d'hydrogène,
2. un atome d'halogène,
3. un groupe alcoxy en C₁-C₃,
4. le groupe nitro,
5. le groupe cyano,
6. un groupe S-alkyle(C₁-C₃),
n est un nombre allant de 1 à 8;
R⁶ représente
1. un atome d'hydrogène,
2. un groupe alkyle en C₁-C₃,
3. un groupe alkylalcényle en C₃-C₅,
4. un groupe alkyl(C₁-C₃)-aryle(C₆C₁₀);
R⁷ représente un atome d'hydrogène ou l'un des radicaux acyle substitués ou non substitués suivants:
alcanoyle en C₁-C₆, alcoxy(C₁-C₃)-alcanoyle(C₂-C₆), alkyl(C₁-C₆)carbamoyl-alcanoyle(C₂-C₆), aryl(C₆-C₁₂)-alcanoyle(C₂-C₆), alcénoyle en C₃-C₇, cycloalkyl(C₃-C₈)carbonyle, cycloalcényl(C₅-C₇)carbonyle, alcoxy(C₁-C₃)carbonyle, aryloxy(C₆-C₁₂)carbonyle, aroyle en C₆-C₁₂, alcoxy(C₁-C₃)-aroyle(C₆-C₁₂), halogénoaroyle en C₆-C₁₂, aryl(C₆-C₁₂)-alcénoyle(C₃-C₆), alcoxy(C₁-C₃)-aryl(C₆-C₁₂)-alcénoyle(C₃-C₆), alkylènedioxy(C₁-C₃)-aryl(C₆-C₁₂)-alcénoyle(C₃-C₆), nitroaryl (C₆-C₁₂)-alcénoyle(C₃-C₆), cyanoaryl(C₆-C₁₂)-alcénoyle(C₃-C₆), halogénoaryl(C₆-C₁₂)-alcénoyle(C₃-C₆), halogénoalkyl(C₁-C₃)-aryl(C₆-C₁₂)-alcénoyle(C₃-C₆), hétérocycloalkyl(C₃-C₈)aryl(C₆-C₁₂)-alcénoyle(C₃-C₆), aminoaryl(C₆-C₁₂)-alcénoyle(C₃-C₆), alkyl(C₁-C₄)aminoaryl(C₆-C₁₂)-alcénoyle(C₃-C₆), acyl(C₂-C₅)amino-aryl(C₆-C₁₂)-cinnamoyle, alcoxy(C₁-C₃)carbonylamino-aryl(C₆-C₁₂)cinnamoyle, alkyl(C₁-C₄)aminocarbonylaminocinnamoyle, hétéroaryl (C₆-C₁₂)-alcanoyl (C₂-C₆)aminoaryl(C₆-C₁₂)-alcénoyle (C₃-C₆), aroyl(C₆-C₁₂)amino-aryl(C₆-C₁₂)-alcénoyle(C₃-C₆), hétéroaryl(C₆-C₁₂)carbonylaminoaryl(C₆-C₁₂)-alcénoyle(C₃-C₆), alkyl(C₁-C₅)-sulfonylamino-aryl(C₆-C₁₂)-alcénoyle(C₃-C₆), alkyl(C₁-C₅)uréido-aryl(C₆-C₁₂)-alcénoyle(C₃-C₆), alcanoyl(C₂-C₆)-aryl(C₆-C₁₂)alcénoyle(C₃-C₆), alcoxy(C₁-C₅)carbonyl-aryl(C₆-C₁₂)-alcénoyle(C₃-C₆), alkyl(C₁-C₅)carbamoyl-aryl(C₆-C₁₂)-alcénoyle(C₃-C₆), aryl(C₆-C₁₂)carbamoylaryl(C₆-C₁₂)-alcénoyle(C₃-C₆), aryl(C₆-C₁₂)-alcoxy(C₁-C₅)carbonyle, alkyl(C₁-C₅)carbamoyle, aryl(C₆-C₁₂)carbamoyle, aroyl(C₆-C₁₂)carbamoyle, alkyl(C₁-C₆)sulfonyle, aryl(C₆-C₁₂)sulfonyle, aryl(C₆-C₁₂)-alkyl(C₁-C₆)sulfonyle et phtaloyle (pour R⁶ = R⁷);
ainsi que leurs sels physiologiquement acceptables, à l'exclusion des composés
8-[2,6-dichloro-3-(2-phtalimidoéthoxy)benzyloxy]-2-méthylquinoléine,
8-[3-(2-aminoéthoxy)-2,6-dichlorobenzyloxy]-2-méthylquinoléine,
8-[3-(2-acétamidoéthoxy)-2,6-dichlorobenzyloxy]-2-méthylquinoléine.

2. Composés de formule (I) selon la revendication 1, dans lesquels les symboles ont les significations suivantes:
R¹, R², R³ sont identiques ou différents et représentent
1. un atome d'hydrogène,
2. un groupe alkyle en C₁-C₃;
R⁴, R⁵ représentent des atomes d'halogène;
R⁶ représente
1. un atome d'hydrogène,
2. le groupe méthyle, éthyle,
3. le groupe benzyle.

3. Composés de formule (I) selon la revendication 1 ou 2, dans laquelle
R⁷ représente
1. un atome d'hydrogène ou
2. un radical acyle qui a la signification indiquée selon la revendication 1.

4. Composés de formule (I) selon les revendications 1 à 3, dans lesquels les symboles ont les significations suivantes:
R¹, R², R³ sont identiques ou différents et représentent
1. un atome d'hydrogène,
2. le groupe méthyle, éthyle, propyle;
R⁴, R⁵ sont des atomes de chlore;
n va de 1 à 4;
R⁶ représente un atome d'hydrogène;
R⁷ représente
1. un atome d'hydrogène,
2. un groupe alcanoyle en C₂-C₅,
3. un groupe alcénoyle en C₃-C₅,
4. un groupe alkyl(C₁-C₅)aminocarbonyle,
5. un groupe aryl(C₆-C₁₀)-alkyl(C₁-C₃)aminocarbonyle,
6. un groupe alkyloxy(C₁-C₅)carbonyle,
7. un groupe aryl(C₆-C₁₀)-alkyloxy(C₁-C₃)carbonyle,
8. un groupe aryl(C₆-C₁₀)-cycloalkyl (C₃-C₇)carbonyle,
9. un reste d'acide *trans*-cinnamique dont le cycle phényle est substitué par jusqu'à 2 radicaux identiques ou différents, choisis dans l'ensemble constitué par
a) un atome d'hydrogène,
b) un groupe alkyle en C₁-C₃,
c) le groupe amino,
d) un groupe mono- ou dialkyl(C₁-C₃)amino,
e) un atome d'halogène,
f) un groupe halogénoalkyle en C₁-C₃,
g) un groupe acyl(C₂-C₅)amino et
h) un groupe alcoxy en C₁-C₃.

5. Procédé pour la préparation des composés de formule (I) selon les revendications 1 à 4, caractérisé en ce que
a) on fait réagir un composé de formule (II) 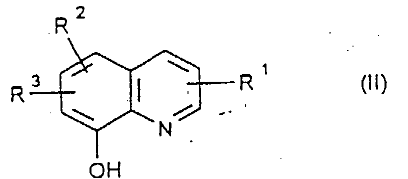 dans laquelle R¹, R² et R³ sont tels que définis plus haut, avec un composé de formule III 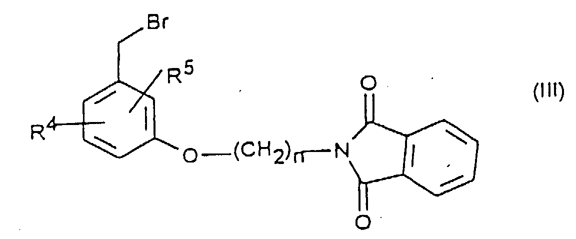 dans laquelle R⁴, R⁵ et n sont tels que définis plus haut, en présence d'hydrures métalliques, tels que le lithium, le potassium ou l'hydrure de sodium, ou de carbonates de métaux alcalins, tels que le carbonate de sodium, potassium ou césium, dans un solvant inerte, tel que le DMF ou le DMSO, à des températures de 0 à 60°C, pour aboutir à un composé de formule (IV) 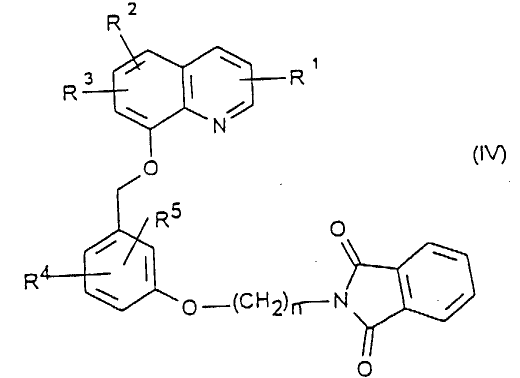 les symboles et variables étant tels que définis plus haut;
b) on convertit un composé de formule (IV) par hydrazinolyse, dans de l'éthanol au reflux, en un composé de formule (Ia) 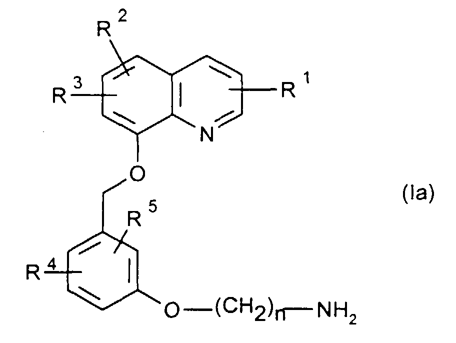 dans laquelle R¹, R², R³, R⁴, R⁵ et n sont tels que définis plus haut;
c) éventuellement on soumet à une acylation et/ou une alkylation les composés de formule (Ia), selon des méthodes connues; et
d) on convertit éventuellement le composés de formule (I) obtenus, selon des méthodes connues, en leurs sels physiologiquement acceptables.

6. Utilisation des composés de formule (I) selon les revendications 1 à 4, en tant que médicament.

7. Médicament, contenant au moins un composé de formule (I) selon les revendications 1 à 4.

## Revendications (Revendications pour l'(les) Etat(s) contractant(s) suivant(s): FI)

1. Composés de formule (I) 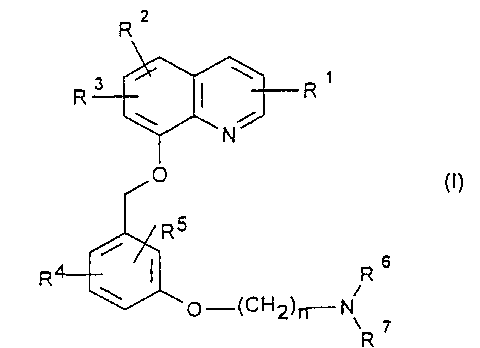 dans laquelle les symboles ont les significations suivantes:
R¹, R², R³ sont identiques ou différents et représentent
1. un groupe alkyle en C₁-C₅,
2. un groupe aryle en C₆-C₁₀,
3. un groupe alkyl(C₁-C₃)-aryle(C₆-C₁₀),
4. un atome d'halogène,
5. un atome d'hydrogène,
6. un groupe cycloalkyle en C₃-C₈,
7. CHO,
8. un groupe CO-O-alkyle(C₁-C₃),
9. COOH;
R⁴, R⁵ sont identiques ou différents et représentent
1. un atome d'hydrogène,
2. un atome d'halogène,
3. un groupe alcoxy en C₁-C₃,
4. le groupe nitro,
5. le groupe cyano,
6. un groupe S-alkyle(C₁-C₃),
n est un nombre allant de 1 à 8;
R⁶ représente
1. un atome d'hydrogène,
2. un groupe alkyle en C₁-C₃,
3. un groupe alkylalcényle en C₃-C₅,
4. un groupe alkyl(C₁-C₃)-aryle(C₆C₁₀);
R⁷ représente un atome d'hydrogène ou l'un des radicaux acyle substitués ou non substitués suivants:
alcanoyle en C₁-C₆, alcoxy(C₁-C₃)-alcanoyle(C₂-C₆), alkyl(C₁-C₆)carbamoyl-alcanoyle(C₂-C₆), aryl(C₆-C₁₂)alcanoyle(C₂-C₆), alcénoyle en C₃-C₇, cycloalkyl(C₃-C₈)carbonyle, cycloalcényl(C₅-C₇)carbonyle, alcoxy(C₁-C₃)carbonyle, aryloxy(C₆-C₁₂)carbonyle, aroyle en C₆-C₁₂, alcoxy(C₁-C₃)-aroyle(C₆-C₁₂), halogénoaroyle en C₆-C₁₂, aryl(C₆-C₁₂)-alcénoyle-(C₃-C₆), alcoxy(C₁-C₃)-aryl(C₆-C₁₂)-alcénoyle(C₃-C₆), alkylènedioxy(C₁-C₃)-aryl(C₆-C₁₂)-alcénoyle(C₃-C₆), nitroaryl(C₆-C₁₂)-alcénoyle(C₃-C₆), cyanoaryl(C₆-C₁₂)-alcénoyle(C₃-C₆), halogénoaryl(C₆-C₁₂)-alcénoyle(C₃-C₆), halogénoalkyl(C₁-C₃)-aryl(C₆-C₁₂)-alcénoyle(C₃-C₆), hétérocycloalkyl(C₃-C₈)aryl(C₆-C₁₂)-alcénoyle(C₃-C₆), aminoaryl(C₆-C₁₂)-alcénoyle(C₃-C₆), alkyl(C₁-C₄)aminoaryl(C₆-C₁₂)-alcénoyle(C₃-C₆), acyl(C₂-C₅)amino-aryl(C₆-C₁₂)-cinnamoyle, alcoxy(C₁-C₃)carbonylamino-aryl(C₆-C₁₂)cinnamoyle, alkyl (C₁-C₄)aminocarbonylaminocinnamoyle, hétéroaryl(C₆-C₁₂)-alcanoyl(C₂-C₆)aminoaryl(C₆-C₁₂)-alcénoyle(C₃-C₆), aroyl(C₆-C₁₂)amino-aryl(C₆-C₁₂)-alcénoyle(C₃-C₆), hétéroaryl(C₆-C₁₂)carbonylaminoaryl(C₆-C₁₂)-alcénoyle(C₃-C₆), alkyl(C₁-C₅)-sulfonylamino-aryl(C₆-C₁₂)-alcénoyle(C₃-C₆), alkyl(C₁-C₅)uréido-aryl(C₆-C₁₂)-alcénoyle(C₃-C₆), alcanoyl(C₂-C₆)-aryl(C₆-C₁₂)alcénoyle (C₃-C₆), alcoxy(C₁-C₅)carbonyl-aryl(C₆-C₁₂)-alcénoyle(C₃-C₆), alkyl(C₁-C₅)-carbamoyl-aryl(C₆-C₁₂)-alcénoyle(C₃-C₆), aryl(C₆-C₁₂)-carbamoylaryl(C₆-C₁₂)-alcénoyle(C₃-C₆), aryl(C₆-C₁₂)-alcoxy(C₁-C₅)carbonyle, alkyl(C₁-C₅)carbamoyle, aryl(C₆-C₁₂)carbamoyle, aroyl(C₆-C₁₂)carbamoyle, alkyl(C₁-C₆)sulfonyle, aryl(C₆-C₁₂)sulfonyle, aryl(C₆-C₁₂)alkyl(C₁-C₆)sulfonyle et phtaloyle (pour R⁶ = R⁷);
ainsi que leurs sels physiologiquement acceptables.

2. Composés de formule (I) selon la revendication 1, dans lesquels les symboles ont les significations suivantes:
R¹, R², R³ sont identiques ou différents et représentent
1. un atome d'hydrogène,
2. un groupe alkyle en C₁-C₃;
R⁴, R⁵ représentent un atome d'halogène;
R⁶ représente
1. un atome d'hydrogène,
2. le groupe méthyle, éthyle,
3. le groupe benzyle.

3. Composés de formule (I) selon la revendication 1 ou 2, dans laquelle
R⁷ représente
1. un atome d'hydrogène ou
2. un radical acyle qui a la signification indiquée selon la revendication 1.

4. Composés de formule (I) selon les revendications 1 à 3, dans lesquels les symboles ont les significations suivantes:
R¹, R², R³ sont identiques ou différents et représentent
1. un atome d'hydrogène,
2. le groupe méthyle, éthyle, propyle;
R⁴, R⁵ sont des atomes de chlore;
n va de 1 à 4;
R⁶ représente un atome d'hydrogène;
R⁷ représente
1. un atome d'hydrogène,
2. un groupe alcanoyle en C₂-C₅,
3. un groupe alcénoyle en C₃-C₅,
4. un groupe alkyl(C₁-C₅)aminocarbonyle,
5. un groupe aryl(C₆-C₁₀)-alkyl(C₁-C₃)aminocarbonyle,
6. un groupe alkyloxy(C₁-C₅)carbonyle,
7. un groupe aryl(C₆-C₁₀)-alkyloxy(C₁-C₃)carbonyle,
8. un groupe aryl(C₆-C₁₀)-cycloalkyl (C₃-C₇)carbonyle,
9. un reste d'acide *trans*-cinnamique dont le cycle phényle est substitué par jusqu'à 2 radicaux identiques ou différents, choisis dans l'ensemble constitué par
a) un atome d'hydrogène,
b) un groupe alkyle en C₁-C₃,
c) le groupe amino,
d) un groupe mono- ou dialkyl(C₁-C₃)amino,
e) un atome d'halogène,
f) un groupe halogénoalkyle en C₁-C₃,
g) un groupe acyl(C₂-C₅)amino et
h) un groupe alcoxy en C₁-C₃.

5. Procédé pour la préparation des composés de formule (I) selon les revendications 1 à 4, caractérisé en ce que
a) on fait réagir un composé de formule (II) 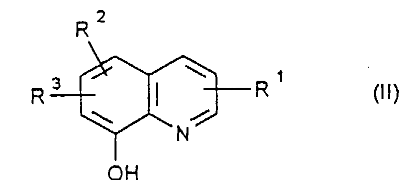 dans laquelle R¹, R² et R³ sont tels que définis plus haut, avec un composé de formule III 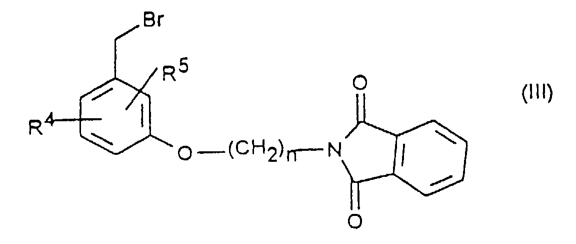 dans laquelle R⁴, R⁵ et n sont tels que définis plus haut, en présence d'hydrures métalliques, tels que le lithium, le potassium ou l'hydrure de sodium, ou de carbonates de métaux alcalins, tels que le carbonate de sodium, potassium ou césium, dans un solvant inerte, tel que le DMF ou le DMSO, à des températures de 0 à 60°C, pour aboutir à un composé de formule (IV) 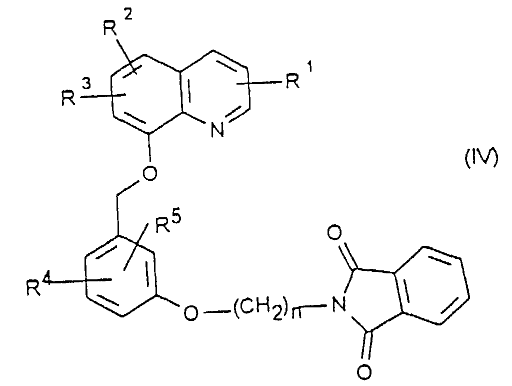 les symboles et variables étant tels que définis plus haut;
b) on convertit un composé de formule (IV) par hydrazinolyse, dans de l'éthanol au reflux, en un composé de formule (Ia) 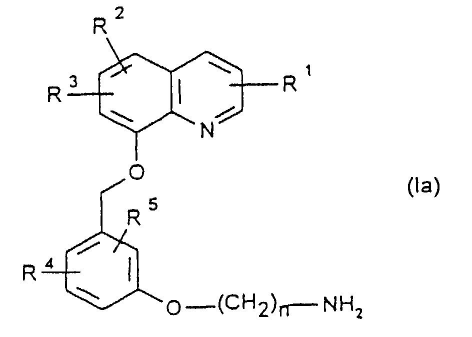 dans laquelle R¹, R², R³, R⁴, R⁵ et n sont tels que définis plus haut;
c) éventuellement on soumet à une acylation et/ou une alkylation les composés de formule (Ia), selon des méthodes connues; et
d) on convertit éventuellement le composés de formule (I) obtenus, selon des méthodes connues, en leurs sels physiologiquement acceptables.

6. Utilisation des composés de formule (I) selon les revendications 1 à 4, en tant que médicament.

7. Médicament, contenant au moins un composé de formule (I) selon les revendications 1 à 4.
